# EUROPÄISCHE PATENTSCHRIFT

(11) **EP 1 596 852 B1**
(45) Veröffentlichungstag und Bekanntmachung des Hinweises auf die Patenterteilung: **27.05.2009**
(21) Anmeldenummer: 04708322.5
(22) Anmeldetag: 05.02.2004
(51) Int. Cl.: A61K 31/195, A61K 31/343, A61P 25/00

(54) **ARZNEIMITTEL ENTHALTEND SUBSTITUIERTE 2-HETEROARYL-AMINOESSIGSÄURE-VERBINDUNGEN**
DRUGS CONTAINING SUBSTITUTED 2-ARYL-AMINOACETIC ACID COMPOUNDS
MEDICAMENTS CONTENANT DES COMPOSES ACIDE 2-HETEROARYLAMINOCETIQUE SUBSTITUE

(30) Priorität: 13.02.2003 DE 10306203
(43) Veröffentlichungstag der Anmeldung: 23.11.2005
(73) Patentinhaber: Grünenthal GmbH, 52078 Aachen (DE)
(72) Erfinder: PRZEWOSNY, Michael, 52064 Aachen (DE); ENGLBERGER, Werner, 52223 Stolberg (DE); SUNDERMANN, Bernd, 52066 Aachen (DE); SCHIENE, Klaus, 40227 Düsseldorf (DE)
(74) Vertreter: Kutzenberger, Helga
(86) Internationale Anmeldenummer: PCT/EP2004/001061
(87) Internationale Veröffentlichungsnummer: WO 2004/071380

(56) Entgegenhaltungen:
- WO-A-02/44171

## Beschreibung

Die vorliegende Erfindung betrifft Arzneimittel enthaltend substituierte 2-Heteroaryl-Aminoessigsäure-Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, weisen jedoch unerwünschte Begleiterscheinungen wie beispielsweise Atemdepression, Erbrechen, Sedierung oder Obstipation oder Toleranzentwicklung auf. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Daneben gibt es weitere Rezeptoren und lonenkanäle, die an dem System der Schmerzentstehung und Schmerzweiterleitung beteiligt sind, wie z. B. der N-Methyl-D-Aspartat (NMDA)-Ionenkanal, über den ein wesentlicher Teil der Kommunikation von Synapsen abläuft und durch den der Calcium-Ionenaustausch zwischen neuronalen Zellen und ihrer Umgebung gesteuert wird.

Kenntnisse über die physiologische Bedeutung von lonenkanal-selektiven Substanzen sind durch die Entwicklung der patch-clamp-Technik gewonnen worden, mit der sich die Wirkung von NMDA-Antagonisten auf den Calciumhaushalt im Zellinneren nachweisen läßt.

WO 02/44171 offenbart Verbindungen ähnlich vorliegender Formel (I), wobei R ⁴ einem Furanyl- oder Thienylrest entspricht. Die Verbindungen sind NMDA- Antagonisten und werden zur Behandlung von Schmerzen eingesetzt.

Eine der vorliegenden Erfindung zugrundeliegende Aufgabe war es daher, neue Arzneimittel zur Verfügung zu stellen, die sich insbesondere zur Bekämpfung von Schmerzen, vorzugsweise von chronischen und/oder neuropathischen Schmerzen eignen und die vorzugsweise die bei den Opioiden auftretenden unerwünschten Begleiterscheinungen nicht oder zumindest im verminderten Umfang zeigen.

Diese Aufgabe wurde durch Bereitstellung eines erfindungsgemäßen Arzneimittels enthaltend wenigstens eine substituierte 2-Heteroaryl-Aminoessigsäure-Verbindung der nachstehenden allgemeinen Formel I gelöst.

Ein Gegenstand der vorliegenden Erfindung ist daher ein Arzneimittel enthaltend wenigstens eine substituierte 2-Heteroaryl-Aminoessigsäure-Verbindung der allgemeinen Formel I, 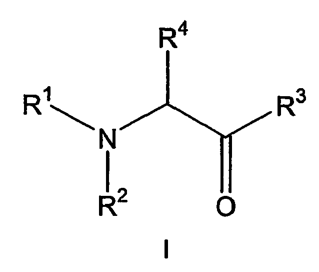 worin
R¹ für einen monocyclischen Aryl- oder Heteroaryl-Rest steht, der wenigstens einfach substituiert und/oder mit einem ggf. wenigstens einfach substituierten, ggf. wenigstens ein Heteroatom als Ringglied aufweisenden, monocyclischen oder polycyklischen Ringsystem kondensiert sein kann,
R² für Wasserstoff oder einen verzweigten oder unverzweigten, ggf. mindestens einfach ungesättigten, ggf. mindestens einfach substituierten aliphatischen Rest steht,
R³ für die Gruppe OR⁵, SR⁵ oder NR⁵R⁶ steht, wobei
   R⁵ und ggf. R⁶, unabhängig voneinander, jeweils für Wasserstoff, einen verzweigten oder unverzweigten, ggf. mindestens einfach ungesättigten, ggf. mindestens einfach substituierten aliphatischen Rest, einen gesättigten oder ungesättigten, ggf. wenigstens ein Heteroatom als Ringglied aufweisenden, ggf. wenigstens einfach substituierten cycloaliphatischen Rest oder für einen ggf. mindestens einfach substituierten Aryl- oder Heteroarylrest stehen, und
R⁴ für einen ggf. mindestens einfach substituierte Benzofuran-oder Benzothiophen-Rest steht,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Unter einem mono- bzw. polycyclischen Ringsystem werden im Sinne der vorliegenden Erfindung mono- bzw. polycyclische Kohlenwasserstoffreste verstanden, die gesättigt, ungesättigt oder aromatisch sein können. Sofern ein polycyclisches Ringsystem vorliegt, kann dieses in verschiedenen Ringen auch zwei oder mehr entsprechende Teilstrukturen unterschiedlichen Sättigungsgrades aufweisen. Ggf. kann das mono- bzw. polycyclische Ringsystem auch ein oder mehrere Heteroatome als Ringglieder aufweisen, wobei die Ringe jeweils gleiche oder unterschiedliche Heteroatome aufweisen können. Sofern ein polycyclisches Ringsystem vorliegt, sind dessen einzelne Ringe vorzugsweise miteinander kondensiert.

Vorzugsweise kommen solche substituierte 2-Heteroaryl-Aminoessigsäure-Verbindungen der vorstehenden allgemeinen Formel I für das erfindungsgemäße Arzneimittel in Betracht, in denen der Rest R¹ für einen 5-oder 6-gliedrigen, monocyclischen Aryl- oder Heteroaryl-Rest steht, der wenigstens einfach substituiert und/oder mit einem ggf. wenigstens einfach substituierten, ggf. wenigstens ein Heteroatom als Ringglied aufweisenden, mono-, bi- oder tricyclischen Ringsystem kondensiert sein kann, wobei die Ringe des Ringsystems jeweils 5 bis 7-gliedrig sind, vorzugsweise für einen ggf. wenigstens einfach substituierten Phenyl-, Naphthyl-, Furanyl-, Thiophenyl-, Pyrrolyl-, Imidazolyl-, Pyrazolyl-, Pyridinyl-, Pyrimidinyl-, Chinolinyl- oder Isochinolinyl-Rest, besonders bevorzugt für einen unsubstituierten oder in 3,5-Position vorzugsweise identisch substituierten Phenyl-Rest steht, und R²-R⁶ die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Ebenfalls bevorzugt kommen solche substituierte 2-Heteroaryl-Aminoessigsäure-Verbindungen der vorstehenden allgemeinen Formel I für das erfindungsgemäße Arzneimittel in Betracht, in denen der Rest R² für Wasserstoff oder einen verzweigten oder unverzweigten, ggf. mindestens einfach ungesättigten, ggf. mindestens einfach substituierten aliphatischen C₁₋₃-Rest, bevorzugt für Wasserstoff, steht, und die Reste R¹ und R³ bis R⁶ jeweils die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Des weiteren bevorzugt kommen solche substituierte 2-Heteroaryl-Aminoessigsäure-Verbindungen der vorstehenden allgemeinen Formel I für das erfindungsgemäße Arzneimittel in Betracht, in denen der Rest R³ für die Gruppen OR⁵, oder NR⁵R⁶, bevorzugt für die Gruppe OR⁵ steht, und die Reste R¹, R² und R⁴ bis R⁶ jeweils die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Weiterhin bevorzugt kommen solche substituierte 2-Heteroaryl-Aminoessigsäure-Verbindungen der vorstehenden allgemeinen Formel I für das erfindungsgemäße Arzneimittel in Betracht, in denen R⁵ und ggf. R⁶ unabhängig voneinander, jeweils für Wasserstoff, einen verzweigten oder unverzweigten, ggf. mindestens einfach ungesättigten, ggf. mindestens einfach substituierten aliphatischen C₁₋₆-Rest stehen, einen gesättigten oder ungesättigten, ggf. wenigstens ein Heteroatom als Ringglied aufweisenden, ggf. wenigstens einfach substituierten cycloaliphatischen C₃₋₈-Rest stehen oder für einen 5- oder 6-gliedrigen, ggf. wenigstens einfach substituierten Aryl- oder Heteroaryl-Rest stehen, bevorzugt für Wasserstoff oder einen ggf. mindestens einfach substituierten unverzweigten aliphatischen C₁₋₆-Rest, besonders bevorzugt für Wasserstoff stehen und die Reste R¹ bis R⁴ jeweils die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Ebenfalls bevorzugt kommen solche substituierte 2-Heteroaryl-Aminoessigsäure-Verbindungen der vorstehenden allgemeinen Formel I in dem erfindungsgemäßen Arzneimittel zum Einsatz, in denen der Rest R⁴ für einen ggf. wenigstens einfach substituierten Benzofuran oder Benzothiophen-Rest, vorzugsweise für einen Rest ausgewählt aus der Gruppe bestehend aus Benzo[b]furan-2-yl, Benzo[b]furan-3-yl, Benzo[b]thiophen-2-yl und Benzo[b]thiophen-3-yl, steht, jeweils unsubstituiert oder wenigstens einfach substituiert, und die Reste R¹ bis R³, R⁵ und R⁶ jeweils die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Sofern R¹ für einen wenigstens einfach substituierten monocyclischen Aryl- oder Heteroaryl-Rest steht und/oder ein wenigstens einfach substituiertes mono- oder polycyclisches Ringsystem aufweist, können die entsprechenden Substituenten, jeweils unabhängig voneinander, bevorzugt ausgewählt werden aus der Gruppe bestehend aus linearem oder verzweigtem, ggf. wenigstens einfach substituiertem, ggf. über ein Sauerstoffatom oder ein Schwefelatom gebundenem C₁₋₆-Alkyl, linearem oder verzweigtem, ggf. wenigstens einfach substituiertem, ggf. über ein Sauerstoffatom oder ein Schwefelatom gebundenem C₂₋₆-Alkenyl, linearem oder verzweigtem, ggf. wenigstens einfach substituiertem, ggf. über ein Sauerstoffatom oder ein Schwefelatom gebundenem C₂₋₆-Alkinyl, ggf. wenigstens einfach substituiertem, ggf. wenigstens ein Heteroatom aufweisenden, ggf. über ein Sauerstoffatom oder ein Schwefelatom gebundenem C₃₋₈-Cycloalkyl, Halogen, OH, SH, CN, ggf. wenigstens einfach substituiertem, ggf. über ein Sauerstoffatom oder ein Schwefelatom gebundenem Phenyl, Naphthyl, Furanyl, Thiophenyl, Pyrrolyl, Imidazolyl, Pyrazolyl, Pyridinyl, Pyrimidinyl, Chinolinyl oder Isochinolinyl. Besonders bevorzugt können die entsprechenden Substituenten ausgewählt werden aus der Gruppe bestehend aus F, Cl, Br, I, OH, SH, unsubstituiertem C₁₋₆-Alkyl, unsubstituiertem C₁₋₆-Alkoxy, C₁₋₆-Perfluoralkyl, C₁₋₆-Perfluoralkoxy, unsubstituiertem C₁₋₆-Alkylsulfanyl, C₁₋₆-Perfluoralkylsulfanyl, unsubstituiertem Phenylsulfanyl, unsubstituiertem Cyclopentyl und unsubstituiertem Cyclohexyl. Ganz besonders bevorzugt können die jeweiligen Substituenten ausgewählt werden aus der Gruppe bestehend aus F, Cl und CF₃.

Sofern einer dieser vorstehend genannten Substituenten selbst einfach oder mehrfach substituiert ist, können dessen Substituenten vorzugsweise ausgewählt werden aus der Gruppe bestehend aus F, Cl, Br, OH, CN, CF₃, CHF₂ und CH₂F.

Sofern R² für einen wenigstens einfach substituierten, verzweigten oder unverzweigten, ggf. mindestens einfach ungesättigten aliphatischen Rest steht, können dessen Substituenten vorzugsweise ausgewählt werden aus der Gruppe bestehend aus F, Cl, Br, OH und CN.

Sofern der monocyklische Heteroarylrest des Restes R⁴ und/oder das ankondensierte monocyklische Ringsystem wenigstens einfach substituiert ist, können die entsprechenden Substituenten, jeweils unabhängig voneinander, bevorzugt ausgewählt werden aus der Gruppe bestehend aus Halogen, CₓF₂ₓ₊₁ (x = ganze zahl von 1-6), CN, OR⁷. SR⁷, CO₂R⁷, CON(R⁷)₂, N(R⁷)₂, ggf. mindestens einfach substituiertem C₃₋₈-Cycloalkyl, ggf. mindestens einfach substituiertem, verzweigtem oder unverzweigtem C₁₋₆-Alkyl, ggf. mindestens einfach substituiertem, verzweigtem oder unverzweigtem C₂₋₆-Alkenyl, ggf. mindestens einfach substituiertem, verzweigtem oder unverzweigtem C₂₋₆-Alkinyl und ggf. mindestens einfach substituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl,
wobei der Rest R⁷ ausgewählt werden kann aus der Gruppe bestehend aus: Wasserstoff, ggf. mindestens einfach substituiertem C₃₋₈-Cycloalkyl, ggf. mindestens einfach substituiertem, verzweigtem oder unverzweigtem C₁₋₆-Alkyl, ggf. mindestens einfach substituiertem, verzweigtem oder unverzweigtem C₂₋₆-Alkenyl, ggf. mindestens einfach substituiertem, verzweigtem oder unverzweigtem C₂₋₆-Alkinyl und ggf. mindestens einfach substituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, bevorzugt aus der Gruppe bestehend aus Wasserstoff, unsubstituiertem C₃₋₈-Cycloalkyl, unsubstituiertem C₁₋₆-Alkyl, C₁₋₆-Perfluoralkoxyl, C₁₋₆-Perfluoralkyl, unsubstituiertem C₂₋₆-Alkenyl, unsubstituiertem C₂₋₆-Alkinyl, und unsubstituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl.

Sofern einer dieser vorstehend genannten aromatischen oder heteroaromatischen Substituenten selbst einfach oder mehrfach substituiert ist, können dessen Substituenten vorzugsweise ausgewählt werden aus der Gruppe bestehend aus F, Cl, Br, OH, CN, CF₃, CHF₂, CH₂F, OCF₃, OCHF₂ und OCH₂F.

Sofern einer dieser vorstehend genannten aliphatischen oder cycloaliphatischen Substituenten selbst einfach oder mehrfach substituiert ist, können dessen Substituenten vorzugsweise ausgewählt werden aus der Gruppe bestehend aus F, Cl, Br, OH und CN.

Sofern R⁵ und/ oder R⁶ für einen mindestens einfach substituierten, verzweigten oder unverzweigten, ggf. mindestens einfach ungesättigten aliphatischen Rest stehen, können dessen Substituenten vorzugsweise ausgewählt werden aus der Gruppe bestehend aus F, Cl, Br, OH und CN.

Sofern R⁵ und/ oder R⁶ für einen mindestens einfach substituierten, gesättigten oder ungesättigten, ggf. wenigstens ein Heteroatom als Ringglied aufweisenden, cycloaliphatischen Rest stehen, können dessen Substituenten vorzugsweise ausgewählt werden aus der Gruppe bestehend aus F, Cl, Br, OH, CN, CF₃, CHF₂, CH₂F, OCF₃, OCHF₂ und OCH₂F.

Sofern R⁵ und/ oder R⁶ für einen mindestens einfach substituierten Aryl- oder Heteroarylrest stehen, können dessen Substituenten vorzugsweise ausgewählt werden aus der Gruppe bestehend aus F, Cl, Br, OH, CN, CF₃, CHF₂, CH₂F, OCF₃, OCHF₂ und OCH₂F

Sofern einer oder mehrere Reste R¹, R⁴, R⁵ oder R⁶ für einen der vorstehend genannten Reste steht, die ein oder mehrere Heteroatome aufweisen, können diese Heteroatome, sofern nicht anders angegeben, vorzugsweise ausgewählt werden aus der Gruppe bestehend aus Stickstoff, Sauerstoff und Schwefel. Besonders bevorzugte Heteroatome sind Sauerstoff und/oder Schwefel.

Ganz besonders bevorzugt enthält das erfindungsgemäße Arzneimittel wenigstens eine substituierte 2-Heteroaryl-Aminoessigsäure-Verbindung der vorstehenden allgemeinen Formel I ausgewählt aus der Gruppe bestehend aus:
Benzo[b]furan-2-yl-(3,5-dichlorphenylamino)-essigsäure,
Benzo[b]furan-2-yl-(3,5-bis(trifluormethyl)phenylamino)-essigsäure,
Benzo[b]thiophen-2-yl-(3,5-dichlorphenylamino)-essigsäure,
Benzo[b]thiophen-3-yl-(3,5-dichlorphenylamino)-essigsäure und
Benzo[b]thiophen-2-yl-(3,5- bis(trifluormethyl)phenylamino-essigsäure,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

Es wurde überraschender Weise gefunden, daß die substituierten 2-Heteroaryl-Aminoessigsäure-Verbindungen der vorstehenden allgemeinen Formel I eine hohe Affinität zur GlycinB-Bindungsstelle des NMDA-Rezeptorkanals aufweisen und sich als GlycinB-Antagonisten am NMDA-Rezeptor Komplex zur Regulation des Calciumhaushalts der Zellen bei der Schmerzweiterleitung und damit unter anderem auch zur Regulation der Schmerzempfindung eignen.

Das erfindungsgemäße Arzneimittel enthaltend wenigstens eine substituierte 2-Heteroaryl-Aminoessigsäure-Verbindung der vorstehenden allgemeinen Formel I zeigt insbesondere eine ausgeprägte Wirksamkeit bei der Bekämpfung von Schmerzen, vorzugsweise von chronischen und/oder neuropathischen Schmerzen. Des weiteren eignet sich das erfindungsgemäße Arzneimittel enthaltend wenigstens eine substituierte 2-Heteroaryl-Aminoessigsäure-Verbindung der vorstehenden allgemeinen Formel I zur Behandlung von Angstzuständen, inflammatorischen und/oder allergischen Reaktionen, Depressionen, Drogen- und/oder Medikamenten- und/oder Alkoholmißbrauch, Gastritis, Diarrhoe, Harninkontinenz, cardiovaskulären Erkrankungen, Atemwegserkrankungen, Husten, seelischen Erkrankungen, Epilepsie, Schizophrenie, neurodegenerativen Erkrankungen, vorzugsweise Morbus Alzheimer und/oder Morbus Huntington und/oder Morbus Parkinson und/oder multipler Sklerose, cerebralen Ischämien, cerebralen Infarkten, Psychosen bedingt durch erhöhten Aminosäurenspiegel, Schlaganfällen, Hirnödernen, Hypoxie, Anoxie, AIDS-Demens, Encephalomyelitis, Tourette-Syndrom, Tinnitus aurium und perinataler Asphyxie.

Die Herstellung der erfindungsgemäß zum Einsatz kommenden substituierten 2-Heteroaryl-Aminoessigsäure-Verbindungen der vorstehenden allgemeinen Formel I kann nach üblichen, dem Fachmann bekannten Verfahren erfolgen, wie z.B. in US 6,232,467; WO 00/24510; WO 01 /55091; N.A. Petasis, I.A. Zavialov, J. Am. Chem. Soc. 119, 445 - 446 (1997); N.A. Petasis, A. Goodman, I.A. Zavialov, Tetrahedron 53, 16463 - 16470 (1997), N.A. Petasis, S. Boral, Tetrahedron Lett. 42, 539 - 542 (2001) und G.S. Currie et al., J. Chem. Soc., Perkin Trans. 1 (2000), 2982 - 2990 beschrieben.

Vorzugsweise wird zur Herstellung der erfindungsgemäß zum Einsatz kommenden substituierten 2-Heteroaryl-Aminoessigsäure-Verbindungen der vorstehenden allgemeinen Formel I wenigstens ein Amin der allgemeinen Formel II 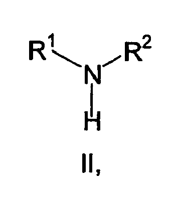 worin R¹ und R² die vorstehend genannte Bedeutung haben, mit Glyoxylsäure (OHC-COOH), ggf. in Form eines Hydrates, und wenigstens einer Boronsäure der allgemeinen Formel III 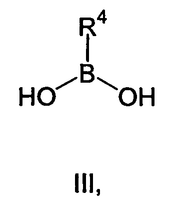 worin R⁴ die vorstehend genannte Bedeutung hat, in einem geeigneten Reaktionsmedium, vorzugsweise in einem organischen Lösungsmittel, besonders bevorzugt in Methylenchlorid oder einem Gemisch enthaltend Methylenchlorid, zu wenigstens einer Verbindung der allgemeinen Formel I ungesetzt und diese ggf. nach üblichen, dem Fachmann bekannten Methoden gereinigt und isoliert.

Die vorstehend beschriebene Umsetzung erfolgt vorzugsweise bei einer Temperatur von 15°C bis 30°C, besonders bevorzugt bei einer Temperatur von 20 bis 25°C.

Das vorstehend beschriebene Verfahren zur Herstellung der in dem erfindungsgemäßen Arzneimittel zum Einsatz kommenden Verbindungen der allgemeinen Formel I hat den Vorteil, daß die eingesetzten Amine der allgemeinen Formel II, die Glyoxylsäurekomponente sowie die Boronsäuren der allgemeinen Formel III in dem Reaktionsmedium löslich, die substituierten 2-Heteroaryl-Aminoessigsäure-Verbindungen der vorstehenden allgemeinen Formel I dagegen üblicherweise unlöslich sind, so daß letztere durch einfache Filtration und Waschen mit dem verwendeten Reaktionsmedium in reiner Form erhalten werden können.

Die so erhaltenen Verbindungen der vorstehenden allgemeinen Formel I, in denen der Rest R³ für die Gruppe OR⁵ steht und R⁵ für Wasserstoff steht, können nach üblichen, dem Fachmann bekannten Verfahren in die entsprechenden Ester, Thioester und Amide überführt werden, in denen der Rest R³ für die Gruppe OR⁵ steht, worin R⁵ die vorstehend genannte Bedeutung mit Ausnahme von Wasserstoff hat, bzw. für die Gruppe SR⁵ oder NR⁵R⁶ steht, worin und R⁵ und ggf. R⁶ jeweils die vorstehend genannte Bedeutung haben.

Die substituierten 2-Heteroaryl-Aminoessigsäure-Verbindungen der vorstehenden allgemeinen Formel I und entsprechende Stereoisomere können nach üblichen, dem Fachmann bekannten Verfahren in Form ihrer physiologisch verträglichen Salze erhalten werden, wobei das erfindungsgemäße Arzneimittel eines oder mehrere Salze einer oder mehrerer Verbindungen der vorstehenden allgemeinen Formel I aufweisen kann.

Bevorzugt sind Salze der Alkalimetalle, Erdalkalimetalle oder Ammoniumsalze, besonders bevorzugt Natrium-, Kalium, Calcium, Magnesium oder Ammoniumsalze, wobei unter Ammoniumsalzen sowohl [NH₄]⁺ Salze als auch [NHₓR₄₋ₓ]⁺ Salze mit R = C₁₋₄-Alkyl und x = 0-3 verstanden werden. Ganz besonders bevorzugt sind Natriumsalze.

Ebenso bevorzugt sind Salze, die beispielsweise durch Umsetzung mit einer anorganischen oder organischen Säure, vorzugsweise mit Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, □-Liponsäure, Acetylglycin, Acetylsalicylsäure, Hippursäure und/oder Asparaginsäure erhalten werden. Ebenfalls ganz besonders bevorzugt sind die Hydrochlorid-Salze der erfindungsgemäß zum Einsatz kommenden Verbindungen der vorstehenden allgemeinen Formel I.

Weiterhin bevorzugt können die entsprechenden Hydrochloridsalze erhalten werden, indem die jeweilige substituierte 2-Heteroaryl-Aminoessigsäure-Verbindung der vorstehenden allgemeinen Formel I und/oder entsprechende Stereoisomere durch Versetzen der in einem geeigneten organischen Lösungsmittel, wie z.B. Butan-2-on (Methylethylketon), gelösten Verbindungen der vorstehenden allgemeinen Formel I oder entsprechender Stereoisomere in unprotonierter Form mit Trimethylsilylchlorid (TMSCI) und Wasser in die entsprechenden Hydrochloridsalze übergeführt wird.

Die substituierten 2-Heteroaryl-Aminoessigsäure-Verbindungen der vorstehenden allgemeinen Formel I und entsprechende Stereoisomere können ggf. nach üblichen, dem Fachmann bekannten Verfahren, ebenso wie die entsprechenden Säuren, die entsprechenden Basen oder die Salze dieser Verbindungen, auch in Form ihrer Solvate, vorzugsweise ihrer Hydrate, erhalten werden.

Sofern die substituierten 2-Heteroaryl-Aminoessigsäure-Verbindungen der vorstehenden allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die substituierten 2-Heteroaryl-Aminoessigsäure-Verbindungen der vorstehenden allgemeinen Formel I und entsprechende Stereoisomere sowie die jeweils entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist die Verwendung wenigstens einer substituierten 2-Heteroaryl-Aminoessigsäure-Verbindung der vorstehenden allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates, zur Herstellung eines Arzneimittels zur Bekämpfung von Schmerzen, vorzugsweise von chronischen und/oder neuropathischen Schmerzen, zur Regulation der GlycinB-Bindungsstelle am NMDA-Rezeptor-Komplex sowie zur Behandlung von Angstzuständen, inflammatorischen und/oder allergischen Reaktionen, Depressionen, Drogen- und/oder Medikamenten- und/oder Alkoholmißbrauch, Gastritis, Diarrhoe, Harninkontinenz, cardiovaskulären Erkrankungen, Atemwegserkrankungen, Husten, seelischen Erkrankungen, Epilepsie, Schizophrenie, neurodegenerativen Erkrankungen, vorzugsweise Morbus Alzheimer und/oder Morbus Huntington und/oder Morbus Parkinson und/oder multipler Sklerose, cerebralen Ischämien, cerebralen Infarkten, Psychosen bedingt durch erhöhten Aminosäurenspiegel, Schlaganfällen, Hirnödemen, Hypoxie, Anoxie, AIDS-Demens, Encephalomyelitis, Tourette-Syndrom, Tinnitus aurium und/oder perinataler Asphyxie.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren substituierten 2-Heteroaryl-Aminoessigsäure-Verbindungen der vorstehenden allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates, enthält das erfindungsgemäße Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Substituierte 2-Heteroaryl-Aminoessigsäure-Verbindungen der vorstehenden allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates, in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 2-Heteroaryl-Aminoessigsäure-Verbindungen der vorstehenden allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates, auch verzögert freisetzen.

Die an den Patienten zu verabreichende Menge der jeweiligen substituierten 2-Heteroaryl-Aminoessigsäure-Verbindungen der vorstehenden allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer substituierten 2-Heteroaryl-Aminoessigsäure-Verbindung der vorstehenden allgemeinen Formel I appliziert, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

### Pharmakologische Methoden

### (a) Rezeptorbindungs-Studie (GlycinB-Bindungsstelle des NMDA-Rezeptorkanals)

Zur Bestimmung der Affinität der erfindungsgemäß zum Einsatz kommenden Verbindungen der vorstehenden allgemeinen Formel I zur Glycin-Bindungsstelle des NMDA-Rezeptorkanals werden an Himmembranhomogenaten (Homogenat von Cortex- und Hippocampus-Areal aus dem Hirn von männlichen Ratten, Stamm Wistar) verwendet, wie beispielsweise in B.M. Baron, B.W. Siegel, B.L. Harrison, R.S. Gross, C. Hawes and P. Towers, Journal of Pharmacology and Experimental Therapeutics, (1996), Vol. 279, S.62 beschrieben.

Hierzu werden Cortex und Hippocampus aus frisch entnommenen Rattengehimen freipräpariert und in 5 mmol/l TRIS-Acetatpuffer, 0,32 mol/l Saccharose pH 7,4 (10 ml/g Frischgewicht) mit einem Potter-Homogenisator (Fa. Braun/Melsungen; 10 Kolbenhübe bei 500 Upm) unter Eiskühlung homogenisiert und anschließend für 10 Minuten bei 1.000 g und 4°C zentrifugiert. Der erste Überstand wird gesammelt und das Sediment erneut mit 5 mmol/l TRIS-Acetatpuffer, 0,32 mol/l Saccharose pH 7,4 (5 ml/g ursprüngliches Frischgewicht) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wird mit dem Überstand aus der ersten Zentrifugation vereinigt und bei 17.000 g für 20 Minuten bei 4°C zentrifugiert. Der Überstand nach dieser Zentrifugation wird verworfen und das Membransediment mit 5 mmol/l TRIS-Acetatpuffer pH 8,0 (20 ml/g ursprüngliches Frischgewicht) aufgenommen und mit 10 Kolbenhüben bei 500 Upm homogenisiert.

Anschließend wird das Membranhomogenat für 1 Stunde bei 4°C inkubiert und für 30 Minuten bei 50.000 g und 4°C zentrifugiert. Der Überstand wird verworfen und das Zentrifugenröhrchen mit dem Membransediment mit Parafilm verschlossen und für 24 Stunden bei -20°C eingefroren. Am folgenden Tag wird das Membransediment aufgetaut und mit eiskaltem 5 mmol/l TRIS-Acetatpuffer, 0,1 % Saponin (Gewicht/Volumen) pH 7,0 (10 ml/g ursprüngliches Frischgewicht) aufgenommen und mit 10 Kolbenhüben bei 500 Upm homogenisiert und anschließend für 20 Minuten bei 50.000 g und 4°C zentrifugiert. Der resultierende Überstand wird verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wird das Membranhomogenat mit 5 mmol/l TRIS-Acetatpuffer pH 7,0 auf eine Proteinkonzentration von 10 mg Protein/ml eingestellt und in Aliquoten bis zur Testung eingefroren.

Für den Rezeptorbindungstest werden Aliquote aufgetaut, 1:10 mit 5 mmol/l TRIS-Acetatpuffer pH 7,0 verdünnt, mit 10 Kolbenhüben bei 500 Upm mit dem Potter-Homogenisator (10 Kolbenhübe bei 500 Upm) unter Eiskühlung homogenisiert und für 60 Minuten bei 55.000 g bei 4°C zentrifugiert. Der Überstand wird dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat werden jeweils 100 µl je 1 ml-Ansatz im Rezeptorbindungstest eingesetzt (0,1 mg Protein/ml im Endansatz). Im Bindungstest wird als Puffer 50 mmol/l TRIS-Acetatpuffer pH 7,0 sowie als radioaktiver Ligand 1 nmol/l (³H)-MDL 105.519 (B.M. Baron et al. 1996) eingesetzt. Der Anteil an unspezifischer Bindung wird in Anwesenheit von 1 mmol/l Glycin bestimmt.

In weiteren Ansätzen werden die erfindungsgemäß zum Einsatz kommenden Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze werden über 120 Minuten bei 4°C inkubiert und anschließend zur Bestimmung des an das Membranhomogenat gebundenen radioaktiven Liganden mittels Filtration durch Glasfaser-Filtermatten (GF/B) geerntet. Die auf den Glasfaser-Filtern zurückgehaltene Radioaktivität wird nach Zugabe von Szintillator (Ready Protein, Fa. Beckmann Coulter GmbH, Krefeld, Deutschland) im β-Counter (Packard TRI-CARB Liquid Szintillation Analzer 2000 CA, Fa. Packard Instrument, Meriden, CT 06450, USA) gemessen.

### (b) Formalin-Test (Maus)

Die Untersuchungen zur Bestimmung der antinozizeptiven Wirkung der erfindungsgemäß zum Einsatz kommenden substituierten 2-Heteroaryl-Aminoessigsäure-Verbindungen der vorstehenden allgemeinen Formel I werden im Formalin-Test an männlichen Albino-Mäusen (NMRI, 25 -35 g, lffa, Credo, Belgien) durchgeführt.

Im Formalin-Test werden die erste (frühe) Phase (0 - 15 min nach Formalin-Injektion) und die zweite (späte) Phase (15 - 60 min nach Formalin-Injektion) unterschieden (D. Dubuisson et. Al., Pain, Vol. 4, pp. 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al, Pain, Vol 52, pp. 259 - 285 (1993)).

Die erfindungsgemäß zum Einsatz kommenden substituierten 2-Heteroaryl-Aminoessigsäure-Verbindungen der vorstehenden allgemeinen Formel I werden in der zweiten Phase des Formalin-Tests untersucht, um Aussagen über Substanzwirkungen im chronisch/ entzündlichen Schmerz zu erhalten.

Durch eine einmalige subkutane Formalin-Injektion (20 µl, 1 %ige wäßrige Lösung) in die dorsale Seite der rechten Hinterpfote wird bei freibeweglichen Versuchstieren eine nozizeptive Reaktion induziert, die sich in deutlichem Lecken und Beißen der betroffenen Pfote äußert.

Für den Untersuchungszeitraum in der zweiten (späten) Phase des Formalin-Tests wird das nozizeptive Verhalten durch Beobachtung der Tiere kontinuierlich erfaßt. Die Quantifizierung des Schmerzverhaltens erfolgt durch Summation der Sekunden, in denen die Tiere im Untersuchungszeitraum Lecken und Beißen der betroffenen Pfote zeigen. Nach Injektion von Substanzen, die im Formalin-Test antinozizeptiv wirksam sind, sind die beschriebenen Verhaltensweisen der Tiere reduziert, evtl. sogar aufgehoben. Entsprechend den Substanzversuchen, bei denen die Tiere Testsubstanz vor Formalin injiziert bekommen, wird den Kontrolltieren Vehikel, d. h. Lösungsmittel (z. B. 0,9 %ige NaCl-Lösung), vor der Formalinapplikation gespritzt. Das Verhalten der Tiere nach Substanzgabe (n=10 pro Substanzdosierung) wird mit einer Kontrollgruppe (n=10) verglichen. Basierend auf der Quantifizierung des Schmerzverhaltens wird die Substanzwirkung im Formalin-Test als Änderung der Kontrolle in Prozent ermittelt. Die ED₅₀-Berechnungen erfolgt mittels Regressionsanalyse. Abhängig von der Applikationsart der erfindungsgemäß zum Einsatz kommenden Verbindungen wird der Applikationszeitpunkt vor der Formalin-Injektion gewählt (intraperitoneal: 15 min, intravenös: 5 min).

Im folgenden wird die Erfindung anhand von Beispielen erläutert.

### Beispiele

Die eingesetzten Chemikalien und Lösungsmittel wurden kommerziell bei den herkömmlichen Anbietern erworben (Acros, Aldrich, Chempur, Fluka, Lancaster und Merck).

Die NMR-Spektren wurden mit NMR-Spektrometem der Firma Bruker Analytik GmbH, Silberstreifen 4, D-76287 Rheinstetten gemessen. Die Gerätebezeichnungen lauten: für 300 MHz: Avance DPX 300 MHz, für 600 MHz: Avance DRX 600 MHz.

Die ESI-Massenspektren wurden mit einem Gerät vom Typ Finnigan LCQ der Firma Thermoquest (Analystische Systeme GmbH, Boschring 12, D-63329 Egelsbach) gemessen und mit der Software Xcalibur ausgewertet.

### Allgemeine Synthesevorschrift zur Herstellung der erfindungsgemäß zum Einsatz kommenden substituierten 2-Heteroaryl-Aminoessigsäure-Verbindungen der vorstehenden allgemeinen Formel I:

In 50 ml Dichlormethan wurden 10 mmol Glyoxylsäurehydrat gelöst, unter Rühren 10 mmol der jeweiligen Amin-Komponente der allgemeinen Formel II und 10 mmol der jeweiligen Boronsäurekomponente der allgemeinen Formel III zugegeben und über Nacht bei Raumtemperatur (ca. 20°C - 25°C) gerührt. Das ausgefallene Reaktionsprodukt wurde abgesaugt, mit wenig kaltem Dichlormethan gewaschen und anschließend im Hochvakuum getrocknet. Es wurden farblose Feststoffe erhalten.

Die jeweils hergestellte Verbindung der vorstehenden allgemeinen Formel I sowie die zu ihrer Herstellung eingesetzten Komponenten der allgemeinen Formeln II und III sind in der nachfolgenden Tabelle 1 wiedergegeben.

**Tabelle 1:**

| **Beispielverbindung** | **Aminkomponente der allgemeinen Formel II** | **Boronsäurekomponente der allgemeinen Formel III** |
|---|---|---|
| Beispiel 1: | | |
| (Benzo[b]furan-2-yl)-(3,5-dichlorphenylamino)-essigsäure | 3,5-Dichlorphenylamin | Benzo[b]furan-2-yl-boronsäure |
| | | |
| Beispiel 2: | 3,5-Dichlorphenylamin | Benzo[b]thiophen-2-yl- |
| (Benzo[b]thiophen-2-yl)-(3,5-dichlorphenylamino)-essigsäure | | boronsäure |
| | | |
| Beispiel 3: | 3,5-Dichlorphenylamin | Benzo[b]thiophen-3-yl- |
| (Benzo[b]thiophen-3-yl)-(3,5-dichlorphenylamino)-essigsäure | | boronsäure |
| | | |
| Beispiel 4: | 3,5-bis-(Trifluormethyl)- | Benzo[b]thiophen-2-yl- |
| (Benzo[b]thiophen-2-yl)-(3,5-bis(trifluormethyl)phenyl)-essigsäure | phenylamin | boronsäure |
| | | |
| Beispiel 5: | 3,5-bis-(Trifluormethyl)- | Benzo[b]furan-2-yl- |
| (Benzo[b]furan-2-yl)-(3,5-bis(trifluormethyl)phenyl)-essigsäure | phenylamin | boronsäure |

### Beispiel 1:

### (Benzo[b]furan-2-yl)-(3,5-dichlorphenylamino)-essigsäure

Ausbeute: 2,03 g (60,4 % der Theorie)
¹H-NMR (d₆-DMSO_{ext.}): δ = 5,57 ppm (d, 1 H, J = 7,5 Hz, α-CH); 6,62 ppm (s, 1 H, Aryl-H); 6,79 ppm (s, 2H, Aryl-H); 6,95 ppm (s, 1H, Aryl-H); 6,98 ppm (d, 1H, J = 8,2 Hz, α-NH); 7,20 - 7,31 ppm (m, 2H, J = 6,4 Hz, Aryl-H); 7,53 ppm (m, 1 H, J = 8,3 Hz, Aryl-H); 7,62 ppm (m, 1 H, J = 8,3 Hz, Aryl-H); 13,40 ppm (s (breit), 1H, CO₂H).
ESI-MS: Molmasse (berechnet für C₁₆H₁₁Cl₂NO₃): 336,18 g/mol
Gemessen (Positiv-Modus): 338,0 (MH⁺); 290,1 (M-CO₂).

### Beispiel 2:

### (Benzo[b]thiophen-2-yl)-(3,5-dichlorphenylamino)-essigsäure

Ausbeute: 3,52 g (100 % der Theorie)
¹H-NMR (d₆-DMSO_{ext.}): δ = 5,67 ppm (d, 1H, J = 7,2 Hz, α-CH); 6,65 ppm (s, 1H, Aryl-H); 6,81 ppm (s, 2H, Aryl-H); 7,09 ppm (d, 1 H, J = 7,5 Hz, α-NH) 7,32 - 7,36 ppm (m, 2H, Aryl-H); 7,53 ppm (s, 1 H, Aryl-H); 7,83 ppm (m, 1 H, J = 6,8 Hz, Aryl-H); 7,89 ppm (m, 1 H, J = 6,5 Hz, Aryl-H); 13,51 ppm (s (breit), 1H, CO₂H).
ESI-MS: Molmasse (berechnet für C₁₆H₁₁Cl₂NO₂S): 352,24 g/mol
Gemessen (Positiv-Modus): 354,0 (MH⁺); 308,2 (M-CO₂).

### Beispiel 3:

### (Benzo[b]thiophen-3-yl)-(3,5-dichlorphenylamino)-essigsäure

Ausbeute: 3,52 g (100 % der Theorie)
¹H-NMR (d₆-DMSO_{ext.}): λ = 5,64 ppm (d, 1H, α-CH); 6,62 ppm (s, 1 H, J = 1,9 Hz, Aryl-H); 6,75 ppm (d, 2H, J = 1,5 Hz, Aryl-H); 7,02 ppm (d, 1 H, J = 7,5 Hz, α-NH); 7,39 - 7,42 ppm (m, 2H, Aryl-H); 7,81 ppm (s, 1 H, Aryl-H); 7,99 ppm (m, 1 H, J = Hz, 7,2 Hz, Aryl-H); 8,07 ppm (m, 1H, J = 7,1 Hz, Aryl-H).
ESI-MS: Molmasse (berechnet für C₁₆H₁₁Cl₂NO₂S): 352,24 g/mol
Gemessen (Positiv-Modus): 351,8 (MH⁺); 306,2 (M-CO₂).

### Beispiel 4:

### (Benzo[b]thiophen-2-yl)-(3,5-bis(trifluormethyl)phenyl)-essigsäure

Ausbeute: 3,83 g (91% der Theorie)
¹H-NMR (d₆-DMSO_{ext.}): λ = 5,91 ppm (d, 1H, J = 7,1 Hz, α-CH); 7,15 ppm (s, 1H, Aryl-H); 7,33 - 7.36 ppm (m, 2H, 2 x Aryl-H); 7,40 ppm (s, 2H, 2 x Aryl-H); 7,49 ppm (d, 1 H, J = 7,6 Hz, α-NH); 7,57 ppm (s, 1H, Aryl-H); 7,84 ppm (d, 1 H, J = 6,7 Hz, Aryl-H); 7,93 ppm (d, 1 H, J = 6,8 Hz, Aryl-H); 13,61 ppm (s (breit), 1H, CO₂H).
ESI-MS: Molmasse (berechnet für C₁₈H₁₁F₆NO₂S): 411,00 g/mol
Gemessen (Negativ-Modus): 419,0 (M-H⁺); 374,4 (M-CO₂).

### Beispiel 5:

### (Benzo[b]furan-2-yl)-(3,5-bis(trifluormethyl)phenyl)-essigsäure

Ausbeute: 1,79 g (32% der Theorie)
¹H-NMR (d₆-DMSO_{ext.}): δ = 5,89 ppm (d, 1 H, J = 7,9 Hz, α-CH); 7,02 ppm (s, 1H, Aryl-H); 7,15 ppm (s (breit), 1 H, α-NH); 7,22 - 7,34 ppm (m, 2H, Aryl-H); 7,42 ppm (s (breit), 3H, Aryl-H); 7,57 ppm (d, 1H, J = 8,3 Hz, Aryl-H); 7,66 ppm (d, 1H, J = 6,8 Hz, Aryl-H); 13,52 ppm (s, breit, 1 H, CO₂H)
ESI-MS: Molmasse (berechnet für C₁₈H₁₁F₆NO₃): 403,26 g/mol
Gemessen (Positiv-Modus): 401,9 (MH⁺); 358,4 (M-CO₂).

### Allgemeine Synthesevorschrift zur Herstellung der entsprechenden Natriumsalze der Verbindungen der vorstehenden allgemeinen Formel I:

10 mmol der jeweiligen substituierten 2-Heteroaryl-Aminoessigsäure-Verbindungen der vorstehenden allgemeinen Formel I wurden in wenig Wasser suspendiert und 10 mmol 1-normaler wäßriger Natronlauge zugegeben. Bei schlechter Löslichkeit wurde soviel Methanol zugetropft, bis vollständige Lösung eintrat. Nach 30 Minuten Rühren bei Raumtemperatur (ca. 20°C-25°) wurde die Reaktionslösung im Rotationsverdampfer eingeengt, die verbliebene Lösung bei -60°C in einem Gemisch aus IsopropanoI/Trockeneis eingefroren und gefriergetrocknet. Die Natriumsalze wurden als farblose Feststoffe erhalten.

### Beispiel 6:

### (Benzofuran-2-yl)-(3,5-dichlorphenylamino)-essigsäure, Natriumsalz

Ausbeute: 3,58 g (100 % der Theorie)
¹H-NMR (d₆-DMSO_{ext.}): δ = 4,66 ppm (m, 1H, α-CH); 6,49 ppm (s, 1H, Aryl-H); 6,53 ppm (m, 1H, α-NH); 6,60 ppm (s, 2H, Aryl-H); 6,68 ppm (s, 1H, Aryl-H); 7,30 - 7,36 ppm (m, 2H, Aryl-H); 7,50 ppm (s, 1H, Aryl-H); 7,81 ppm (d, 1H, J = 7,5 Hz, Aryl-H); 7,89 ppm (1 H, d, J = 7,5 Hz, Aryl-H).

### Beispiel 7:

### (Benzo[b]thiophen-2-yl)-(3,5-dichlorphenylamino)-essigsäure, Natriumsalz

Ausbeute: 3,27 g (87,3% der Theorie)
¹H-NMR (d₆-DMSO_{ext.}): δ= 5,59 ppm (d, 1H, J = 5,3 Hz, α-CH); 6,64 ppm (s, 1H, Aryl-H); 6,79 ppm (s, 2H, Aryl-H); 7,03 ppm (d, 1H, J (= 7,6 Hz, α-NH); 7,30 - 7,36 ppm (m, 2H, Aryl-H); 7,50 ppm (s, 1H, Aryl-H); 7,85 ppm (m, 1H, Aryl-H); 7,90 ppm (m, 1H, Aryl-H).

### Beispiel 8:

### (Benzo[b]thiophen-3-yl)-(3,5-dichlorphenylamino)-essigsäure, Natriumsalz

Ausbeute: 3,54 g (94,5 % der Theorie)
¹H-NMR (d₆-DMSO_{ext.}): δ = 5,65 ppm (d, 1H, α-CH); 6,55 ppm (m, 1H, Aryl-H); 6,65 ppm (s, 1H, Aryl-H); 6,80 ppm (s, 2H, Aryl-H); 7,05 ppm (d, 1H, α-NH); 7,30 - 7,45 ppm (m, 2H, Aryl-H); 7,80 ppm (s, 1H, Aryl-H); 8,05 ppm (m, 1H, Aryl-H); 8,10 ppm (m, 1H, Aryl-H).

### Beispiel 9:

### (Benzo[b]thiophen-2-yl)-(3,5-bis(trifluormethyl)phenyl)-essigsäure, Natriumsalz

Ausbeute: 4,41 g (100% der Theorie)
¹H-NMR (d₆-DMSO_{ext.}): δ = 4,92 ppm (m, 1 H, α-CH); 6,98 ppm (s, 1H, Aryl-H); 7,14 ppm (d, 1H, J = 4,5 Hz, α-NH); 7,20 - 7,24 ppm (m, 3H, 3 x Aryl-H); 7,27 ppm (t, 1H, J = 7,6 Hz, Aryl-H); 7,33 ppm (s, 1H, Aryl-H); 7,70 ppm (d, 1H, J = 8,3 Hz, Aryl-H); 7,89 ppm (d, 1H, J = 8,3 Hz, Aryl-H).
ESI-MS: Molmasse (berechnet für C₁₈H₁₀F₆NNaO₂S): 441,02 g/mol
Gemessen (Negativ-Modus): 419,0 (M-Na⁺); 374,4 (M-Na⁺-CO₂).

### Beispiel 10:

### (Benzo[b]furan-2-yl)-(3,5-bis(trifluormethyl)phenyl)-essigsäure, Natriumsalz

Ausbeute: 3,85 g (90.5% der Theorie)
¹H-NMR (d₆-DMSO_{ext.}): δ = 4,80 ppm (s, 1H, α-CH); 6,72 ppm (s, 1H, Aryl-H); 6,95 ppm (s, 1H, Aryl-H); 6,98 ppm (d, 1H, J = 4,5 Hz, α-NH);
7,14 - 7,19 ppm (m, 2H, J = 7,5 Hz, 2 x Aryl-H); 7,21 ppm (s, 2H, 2 x Aryl-H); 7,45 ppm (d, 1H, J = 8,3 Hz, Aryl-H); 7,52 ppm (d, 1H, J = 7,6 Hz, Aryl-H).
ESI-MS: Molmasse (berechnet für C₁₈H₁₀F₆NNaO₃): 425,28 g/mol
Gemessen (Negativ-Modus): 401,9 (M-Na⁺); 358,5 (M-Na⁺-CO₂).

### Allgemeine Synthesevorschrift zur Herstellung der entsprechenden Hydrochloridsalze der Verbindungen der vorstehenden allgemeinen Formel I:

10 mmol der jeweiligen substituierten 2-Heteroaryl-Aminoessigsäure-Verbindung der vorstehenden allgemeinen Formel I wurden in 20 ml Butanon gelöst, unter Schutzgasatmosphäre z. B. Stickstoff in einem Eisbad abgekühlt, 10 mmol Trimethylsllychlorid und in einem Schuß 10 mmol Wasser zugegeben. Nach Rühren über Nacht wurde der ausgefallene Feststoff abfiltriert, mit wenig Butanon und anschließend mit Diethylether gewaschen. Die Hydrochloridsalze wurden als farblose Feststoffe erhalten.

### Beispiel 11:

### (Benzo[b]thiophen-2-yl)-(3,5-dichlorphenylamino)-essigsäure, Hydrochlorid

Ausbeute: 2,02 g (51,9 % der Theorie)
¹H-NMR (d₆-DMSO_{ext.}): δ = 5,63 ppm (m, 1H, J = 7,1 Hz, α-CH); 6,63 ppm (s, 1H, Aryl-H); 6,79 ppm (s, 2H, 2 x Aryl-H); 7,05 ppm (d, 1H, J = 7,5 Hz, α-NH); 7,31 - 7,35 ppm (m, 2H, 2 x Aryl-H); 7,52 ppm (s, 1H, Aryl-H); 7,81 ppm (m, 1H, J = 9,0 Hz, Aryl-H); 7,88 ppm (m, 1H, J = 8,6 Hz, Aryl-H); 13,48 ppm (s (breit), 1H, CO₂H).
ESI-MS: Molmasse (berechnet für C₁₆H₁₁Cl₂NO₂S): 349,00 g/mol
Gemessen (Positiv-Modus): 353,9 (MH⁺-HCl); 306,1 (M-HCl-CO₂).

### Beispiel 12:

### (Benzo[b]thiophen-3-yl)-(3,5-dichlorphenylamino)-essigsäure, Hydrochlorid

Ausbeute: 3,25 g (87,3 % der Theorie)
¹H-NMR (d₆-DMSOₑₓₜ): δ = 5,66 ppm (s (breit), 1H, α-CH); 6,65 ppm (s, 1H, Aryl-H); 6,77 ppm (s, 2H, Aryl-H); 7,04 ppm (m, 2H, 2 x Aryl-H); 7,37 - 7,45 ppm (m, 2H, 2 x Aryl-H); 7,82 ppm (s, 1H, Aryl-H); 7,82 ppm (s, 1H, Aryl-H); 8,00 ppm (m, 1H, J = 8,7 Hz, Aryl-H); 8,07 ppm (m, 1 H, J = 7,2 Hz, Aryl-H); 13,19 ppm (s (breit), 1H, CO₂H).

### Pharmakologische Daten

### (a) Rezeptorbindungs-Studie (Glycin-Bindungsstelle des NMDA-Rezeptorkanals)

Die Affinität der erfindungsgemäß zum Einsatz kommenden Verbindungen der vorstehenden allgeimen Formel I zur GlycinB-Bindungsstelle des NMDA-Rezeptorkanals wurde wie vorstehend beschrieben ermittelt. Die untersuchten Verbindungen zeigten jeweils eine ausgeprägte Affinität zu der GlycinB-Bindungsstelle des NMDA-Rezeptor-kanals. Die Werte einiger ausgewählter Verbindungen sind in der nachfolgenden Tabelle 2 jeweils als prozentualer Anteil des gebundenen radioaktiven Liganden angegeben, der bei einer Konzentration von 10µM der zu prüfenden Verbindung aus seiner spezifischen Bindung verdrängt wird.

**Tabelle 2:**

| **Verbindung gemäß Beispiel** | **% Hemmung** |
|---|---|
| 2 | 85 |
| 3 | 15 |
| 6 | 97 |
| 7 | 85 |
| 8 | 24 |
| 9 | 13 |
| 10 | 29 |

### (b) Formalin-Test (Maus)

Die analgetische Wirkung der erfindungsgemäß zum Einsatz kommenden Verbindungen wurde wie vorstehend beschrieben ermittelt.

Die untersuchten Verbindungen zeigten eine mittelstarke bis starke Hemmung der durch Formalin induzierten Nozizeption.

In der nachfolgenden Tabelle sind die Werte einiger ausgewählter Verbindungen wiedergegeben.

**Tabelle 3:**

| **Verbindung** | **Hemmung (%) bei Dosis** |
|---|---|
| 2 | 55% (bei 46,4 mg/kg) |
| 6 | 42% (bei 10 mg/kg) |
| 7 | 11 % (bei 46,4 mg/kg) |
| 10 | 60% (bei 46,4 mg/kg) |

## Patentansprüche

1. Arzneimittel enthaltend wenigstens eine substituierte 2-Heteroaryl-Aminoessigsäure-Verbindung der allgemeinen Formel I, 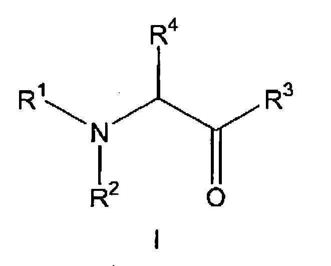 worin
R¹ für einen monocyclischen Aryl- oder Heteroaryl-Rest steht, der wenigstens einfach substituiert und/oder mit einem ggf. wenigstens einfach substituierten, ggf. wenigstens ein Heteroatom als Ringglied aufweisenden, monocyclischen oder polycyklischen Ringsystem kondensiert sein kann, wobei
die entsprechenden Substituenten, jeweils unabhängig voneinander, ausgewählt werden aus der Gruppe bestehend aus linearem oder verzweigtem, ggf. wenigstens einfach substituiertem, ggf. über ein Sauerstoffatom oder ein Schwefelatom gebundenem C₁₋₆-Alkyl, linearem oder verzweigtem, ggf. wenigstens einfach substituiertem, ggf. über ein Sauerstoffatom oder ein Schwefelatom gebundenem C₂₋₆-Alkenyl, linearem oder verzweigtem, ggf. wenigstens einfach substituiertem, ggf. über ein Sauerstoffatom oder ein Schwefelatom gebundenem C₂₋₆-Alkinyl, ggf. wenigstens einfach substituiertem, ggf. wenigstens ein Heteroatom aufweisenden, ggf. über ein Sauerstoffatom oder ein Schwefelatom gebundenem C₃₋₈-Cycloalkyl, Halogen, OH, SH, CN, ggf. wenigstens einfach substituiertem, ggf. über ein Sauerstoffatom oder ein Schwefelatom gebundenem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, wobei
sofern einer dieser vorstehend genannten Substituenten selbst einfach oder mehrfach substituiert ist, werden dessen Substituenten ausgewählt aus der Gruppe bestehend aus F, Cl, Br, OH, CN, CF₃, CHF₂ und CH₂F,
R² für Wasserstoff oder einen verzweigten oder unverzweigten, ggf. mindestens einfach ungesättigten, ggf. mindestens einfach substituierten aliphatischen Rest steht, wobei
dessen Substituenten ausgewählt werden aus der Gruppe bestehend aus F, Cl, Br, OH und CN,
R³ für die Gruppe OR⁵, SR⁵ oder NR⁵R⁶ steht, wobei
R⁵ und ggf. R⁶, unabhängig voneinander, jeweils für Wasserstoff, einen verzweigten oder unverzweigten, ggf. mindestens einfach ungesättigten, ggf. mindestens einfach substituierten aliphatischen Rest steht oder einen gesättigten oder ungesättigten, ggf. wenigstens ein Heteroatom als Ringglied aufweisenden, ggf. wenigstens einfach substituierten cycloaliphatischen Rest oder für einen ggf. mindestens einfach substituierten Aryl- oder Heteroarylrest steht, wobei
sofern R⁵ und/ oder R⁶ für einen mindestens einfach substituierten, verzweigten oder unverzweigten, ggf. mindestens einfach ungesättigten aliphatischen Rest stehen, dessen Substituenten ausgewählt werden aus der Gruppe bestehend aus F, Cl, Br, OH und CN,
sofern R⁵ und/ oder R⁶ für einen mindestens einfach substituierten, gesättigten oder ungesättigten, ggf. wenigstens ein Heteroatom als Ringglied aufweisenden, cycloaliphatischen Rest stehen, dessen Substituenten ausgewählt werden aus der Gruppe bestehend aus F, Cl, Br, OH, CN, CF₃, CHF₂, CH₂F, OCF₃, OCHF₂ und OCH₂F,
sofern R⁵ und/ oder R⁶ für einen mindestens einfach substituierten Aryl- oder Heteroarylrest stehen, dessen Substituenten ausgewählt werden aus der Gruppe bestehend aus F, Cl, Br, OH, CN, CF₃, CHF₂, CH₂F, OCF₃, OCHF₂ und OCH₂F,
und
R⁴ für einen ggf. wenigstens einfach substituierten Benzofuran- oder Benzothiophen-Rest steht, wobei
die entsprechenden Substituenten, jeweils unabhängig voneinander, ausgewählt werden aus der Gruppe bestehend aus Halogen, CₓF₂ₓ₊₁ (x = ganze Zahl von 1-6), CN, OR⁷. SR⁷, CO₂R⁷, CON(R⁷)₂, N(R⁷)₂, ggf. mindestens einfach substituiertem C₃₋₈-Cycloalkyl, ggf. mindestens einfach substituiertem, verzweigtem oder unverzweigtem C₁₋₆-Alkyl, ggf. mindestens einfach substituiertem, verzweigtem oder unverzweigtem C₂₋₆-Alkenyl, ggf. mindestens einfach substituiertem, verzweigtem oder unverzweigtem C₂₋₆-Alkinyl und ggf. mindestens einfach substituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, wobei
der Rest R⁷ ausgewählt wird aus der Gruppe bestehend aus Wasserstoff, ggf. mindestens einfach substituiertem C₃₋₈-Cycloalkyl, ggf. mindestens einfach substituiertem, verzweigtem oder unverzweigtem C₁₋₆-Alkyl, ggf. mindestens einfach substituiertem, verzweigtem oder unverzweigtem C₂₋₆-Alkenyl, ggf. mindestens einfach substituiertem, verzweigtem oder unverzweigtem C₂₋₆-Alkinyl und ggf. mindestens einfach substituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, wobei
sofern einer dieser vorstehend genannten aromatischen oder heteroaromatischen Substituenten selbst einfach oder mehrfach substituiert ist, dessen Substituenten ausgewählt werden aus der Gruppe bestehend aus F, Cl, Br, OH, CN, CF₃, CHF₂, CH₂F, OCF₃, OCHF₂ und OCH₂F,
sofern einer dieser vorstehend genannten aliphatischen oder cycloaliphatischen Substituenten selbst einfach oder mehrfach substituiert ist, dessen Substituenten ausgewählt werden aus der Gruppe bestehend aus F, Cl, Br, OH und CN,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

2. Arzneimittel gemäß Anspruch 1, **dadurch gekennzeichnet, daß** der Rest R¹ für einen 5-oder 6-gliedrigen, monocyclischen Aryl- oder Heteroaryl-Rest steht, der wenigstens einfach substituiert und/oder mit einem ggf. wenigstens einfach substituierten, ggf. wenigstens ein Heteroatom als Ringglied aufweisenden, mono-, bi- oder tricyclischen Ringsystem kondensiert sein kann, wobei die Ringe des Ringsystems jeweils 5 bis 7-gliedrig sind, vorzugsweise für einen ggf. wenigstens einfach substituierten Phenyl-, Naphthyl-, Furanyl-, Thiophenyl-, Pyrrolyl-, Imidazolyl-, Pyrazolyl-, Pyridinyl-, Pyrimidinyl-, Chinolinyl- oder Isochinolinyl-Rest, besonders bevorzugt für einen unsubstituierten oder in 3,5-Position vorzugsweise identisch substituierten Phenyl-Rest steht, wobei
die entsprechenden Substituenten, jeweils unabhängig voneinander, ausgewählt werden aus der Gruppe bestehend aus linearem oder verzweigtem, ggf. wenigstens einfach substituiertem, ggf. über ein Sauerstoffatom oder ein Schwefelatom gebundenem C₁₋₆-Alkyl, linearem oder verzweigtem, ggf. wenigstens einfach substituiertem, ggf. über ein Sauerstoffatom oder ein Schwefelatom gebundenem C₂₋₆-Alkenyl, linearem oder verzweigtem, ggf. wenigstens einfach substituiertem, ggf. über ein Sauerstoffatom oder ein Schwefelatom gebundenem C₂₋₆-Alkinyl, ggf. wenigstens einfach substituiertem, ggf. wenigstens ein Heteroatom aufweisenden, ggf. über ein Sauerstoffatom oder ein Schwefelatom gebundenem C₃₋₈-Cycloalkyl, Halogen, OH, SH, CN, ggf. wenigstens einfach substituiertem, ggf. über ein Sauerstoffatom oder ein Schwefelatom gebundenem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, wobei
sofern einer dieser vorstehend genannten Substituenten selbst einfach oder mehrfach substituiert ist, dessen Substituenten ausgewählt werden aus der Gruppe bestehend aus F, Cl, Br, OH, CN, CF₃, CHF₂ und CH₂F.

3. Arzneimittel gemäß Anspruch 1 oder 2, **dadurch gekennzeichnet, daß** der Rest R² für Wasserstoff oder einen verzweigten oder unverzweigten, ggf. mindestens einfach ungesättigten, ggf. mindestens einfach substituierten aliphatischen C₁₋₃-Rest, bevorzugt für Wasserstoff, steht, wobei
dessen Substituenten ausgewählt werden aus der Gruppe bestehend aus F, Cl, Br, OH und CN.

4. Arzneimittel gemäß einem der Ansprüche 1 bis 3, **dadurch gekennzeichnet, daß** der Rest R³ für die Gruppe OR⁵ oder NR⁵R⁶, bevorzugt für die Gruppe OR⁵, steht.

5. Arzneimittel gemäß einem der Ansprüche 1 bis 4, **dadurch gekennzeichnet, daß** die Reste R⁵ und ggf. R⁶, unabhängig voneinander, jeweils für Wasserstoff, für einen verzweigten oder unverzweigten, ggf. mindestens einfach ungesättigten, ggf. mindestens einfach substituierten aliphatischen C₁₋₆-Rest stehen, für einen gesättigten oder ungesättigten, ggf. wenigstens ein Heteroatom als Ringglied aufweisenden, ggf. wenigstens einfach substituierten cycloaliphatischen C₃₋₈-Rest stehen oder für einen 5- oder 6-gliedrigen, ggf. wenigstens einfach substituierten Aryl- oder Heteroaryl-Rest stehen, bevorzugt für Wasserstoff oder einen ggf. mindestens einfach substituierten aliphatischen C₁₋₆-Rest, besonders bevorzugt für Wasserstoff stehen, wobei
sofern R⁵ und/ oder R⁶ für einen mindestens einfach substituierten, verzweigten oder unverzweigten, ggf. mindestens einfach ungesättigten aliphatischen Rest stehen, dessen Substituenten ausgewählt werden aus der Gruppe bestehend aus F, Cl, Br, OH und CN,
sofern R⁵ und/ oder R⁶ für einen mindestens einfach substituierten, gesättigten oder ungesättigten, ggf. wenigstens ein Heteroatom als Ringglied aufweisenden, cycloaliphatischen Rest stehen, dessen Substituenten ausgewählt werden aus der Gruppe bestehend aus F, Cl, Br, OH, CN, CF₃, CHF₂, CH₂F, OCF₃, OCHF₂ und OCH₂F,
sofern R⁵ und/ oder R⁶ für einen mindestens einfach substituierten Aryl- oder Heteroarylrest stehen, dessen Substituenten ausgewählt werden aus der Gruppe bestehend aus F, Cl, Br, OH, CN, CF₃, CHF₂, CH₂F, OCF₃, OCHF₂ und OCH₂F.

6. Arzneimittel gemäß einem der Ansprüche Anspruch 1 bis 5, **dadurch gekennzeichnet, daß** der Rest R⁴ für einen Rest ausgewählt aus der Gruppe bestehend aus Benzo[b]furan-2-yl, Benzo[b]furan-3-yl, Benzo[b]thio-phen-2-yl und Benzo[b]thiophen-3-yl, jeweils unsubstituiert oder wenigstens einfach substituiert, steht, wobei
die entsprechenden Substituenten, jeweils unabhängig voneinander, ausgewählt werden aus der Gruppe bestehend aus Halogen, CₓF₂ₓ₊₁ (x = ganze Zahl von 1-6), CN, OR⁷. SR⁷, CO₂R⁷, CON(R⁷)₂, N(R⁷)₂, ggf. mindestens einfach substituiertem C₃₋₈-Cycloalkyl, ggf. mindestens einfach substituiertem, verzweigtem oder unverzweigtem C₁₋₆-Alkyl, ggf. mindestens einfach substituiertem, verzweigtem oder unverzweigtem C₂₋₆-Alkenyl, ggf. mindestens einfach substituiertem, verzweigtem oder unverzweigtem C₂₋₆-Alkinyl und ggf. mindestens einfach substituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, wobei
der Rest R⁷ ausgewählt wird aus der Gruppe bestehend aus Wasserstoff, , ggf. mindestens einfach substituiertem C₃₋₈-Cycloalkyl, ggf. mindestens einfach substituiertem, verzweigtem oder unverzweigtem C₁₋₆-Alkyl, ggf. mindestens einfach substituiertem, verzweigtem oder unverzweigtem C₂₋₆-Alkenyl, ggf. mindestens einfach substituiertem, verzweigtem oder unverzweigtem C₂₋₆-Alkinyl und ggf. mindestens einfach substituiertem Phenyl, Naphthyl, Furanyl, Thiophenyl, Pyrrolyl, Imidazolyl, Pyrazolyl, Pyridinyl, Pyrimidinyl, Chinolinyl oder Isochinolinyl, wobei
sofern einer dieser vorstehend genannten aromatischen oder heteroaromatischen Substituenten selbst einfach oder mehrfach substituiert ist, können dessen Substituenten vorzugsweise ausgewählt werden aus der Gruppe bestehend aus F, Cl, Br, OH, CN, CF₃, CHF₂, CH₂F, OCF₃, OCHF₂ und OCH₂F,
sofern einer dieser vorstehend genannten aliphatischen oder cycloaliphatischen Substituenten selbst einfach oder mehrfach substituiert ist, können dessen Substituenten vorzugsweise ausgewählt werden aus der Gruppe bestehend aus F, Cl, Br, OH und CN.

7. Arzneimittel gemäß einem der Ansprüche 1 bis 6, **dadurch gekennzeichnet, daß** die Verbindung der allgemeinen Formel I ausgewählt ist aus der Gruppe bestehend aus:
Benzo[b]furan-2-yl-(3,5-dichlorphenylamino)-essigsäure,
Benzo[b]furan-2-yl-(3,5-bis(trifluormethyl)phenylamino)-essigsäure,
Benzo[b]thiophen-2-yl-(3,5-dichlorphenylamino)-essigsäure,
Benzo[b]thiophen-3-yl-(3,5-dichlorphenylamino)-essigsäure und
Benzo[b]thiophen-2-yl-(3,5-bis(trifluormethyl)phenylamino-essigsäure,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer Säure oder ihrer Base oder in Form ihres physiologisch verträglichen Salzes, insbesondere Natriumsalzes oder Hydrochloridsalzes, oder jeweils in Form ihres Solvates, insbesondere Hydrates.

8. Arzneimittel gemäß einem oder mehreren der Ansprüche 1 bis 7 zur Bekämpfung von Schmerzen.

9. Arzneimittel gemäß Anspruch 8 zur Bekämpfung chronischer Schmerzen.

10. Arzneimittel gemäß Anspruch 8 zur Bekämpfung neuropathischer Schmerzen.

11. Arzneimittel gemäß einem oder mehreren der Ansprüche 1 bis 7 zur Anxiolyse.

12. Arzneimittel gemäß einem oder mehreren der Ansprüche 1 bis 7 zur Behandlung von inflammatorischen und/oder allergischen Reaktionen.

13. Arzneimittel gemäß einem oder mehreren der Ansprüche 1 bis 7 zur Behandlung von Depressionen.

14. Arzneimittel gemäß einem oder mehreren der Ansprüche 1 bis 7 zur Behandlung bei Drogen- und/oder Medikamenten und/oder Alkoholmißbrauch.

15. Arzneimittel gemäß einem oder mehreren der Ansprüche 1 bis 7 zur Behandlung von Gastritis.

16. Arzneimittel gemäß einem oder mehreren der Ansprüche 1 bis 7 zur Behandlung von Diarrhoe.

17. Arzneimittel gemäß einem oder mehreren der Ansprüche 1 bis 7 zur Behandlung von Harninkontinenz.

18. Arzneimittel gemäß einem oder mehreren der Ansprüche 1 bis 7 zur Behandlung von cardiovaskulären Erkrankungen.

19. Arzneimittel gemäß einem oder mehreren der Ansprüche 1 bis 7 zur Behandlung von Atemwegserkrankungen.

20. Arzneimittel gemäß einem oder mehreren der Ansprüche 1 bis 7 zur Behandlung von Husten.

21. Arzneimittel gemäß einem oder mehreren der Ansprüche 1 bis 7 zur Behandlung von seelischen Erkrankungen.

22. Arzneimittel gemäß einem oder mehreren der Ansprüche 1 bis 7 zur Behandlung von Epilepsie.

23. Arzneimittel gemäß einem oder mehreren der Ansprüche 1 bis 7 zur Behandlung von Schizophrenie.

24. Arzneimittel gemäß einem oder mehreren der Ansprüche 1 bis 7 zur Behandlung von neurodegenerativen Erkrankungen, vorzugsweise Morbus'Alzheimer und/oder Morbus Huntington und/oder Morbus Parkinson und/oder multipler Sklerose.

25. Arzneimittel gemäß einem oder mehreren der Ansprüche 1 bis 7 zur Behandlung von cerebralen lschämien.

26. Arzneimittel gemäß einem oder mehreren der Ansprüche 1 bis 7 zur Behandlung cerebraler Infarkte.

27. Arzneimittel gemäß einem oder mehreren der Ansprüche 1 bis 7 zur Behandlung von Psychosen bedingt durch erhöhten Aminosäurespiegel.

28. Arzneimittel gemäß einem oder mehreren der Ansprüche 1 bis 7 zur Behandlung von Schlaganfällen.

29. Arzneimittel gemäß einem oder mehreren der Ansprüche 1 bis 7 zur Behandlung von Himödemen.

30. Arzneimittel gemäß einem oder mehreren der Ansprüche 1 bis 7 zur Behandlung von Hypoxie.

31. Arzneimittel gemäß einem oder mehreren der Ansprüche 1 bis 7 zur Behandlung von Anoxie.

32. Arzneimittel gemäß einem oder mehreren der Ansprüche 1 bis 7 zur Behandlung von AIDS-Demens.

33. Arzneimittel gemäß einem oder mehreren der Ansprüche 1 bis 7 zur Behandlung von Encephalomyelitis.

34. Arzneimittel gemäß einem oder mehreren der Ansprüche 1 bis 7 zur Behandlung des Tourette-Syndroms.

35. Arzneimittel gemäß einem oder mehreren der Ansprüche 1 bis 7 zur Behandlung von Tinnitus aurium.

36. Arzneimittel gemäß einem oder mehreren der Ansprüche 1 bis 7 zur Behandlung bei perinataler Asphyxie.

37. Verwendung wenigstens einer substituierten 2-Heteroaryl-Aminoessigsäure-Verbindung gemäß einem oder mehreren der Ansprüche 1 bis 7 zur Herstellung eines Arzneimittels zur Behandlung von Schmerzen, vorzugsweise von chronischen Schmerzen und/oder neuropathischen Schmerzen.

38. Verwendung wenigstens einer Verbindung gemäß einem oder mehreren der Ansprüche 1 bis 7 zur Herstellung eines Arzneimittels zur Behandlung von Angstzuständen, inflammatorischen und/ oder allergischen Reaktionen, Depressionen, Drogen- und/oder Medikamenten- und/oder Alkoholmißbrauch, Gastritis, Diarrhoe, Harninkontinenz, cardiovaskulären Erkrankungen, Atemwegserkrankungen, Husten, seelischen Erkrankungen, Epilepsie, Schizophrenie, neurodegenerativen Erkrankungen, vorzugsweise Morbus Alzheimer und/oder Morbus Huntington und/ oder Morbus Parkinson und/oder multipler Sklerose, cerebralen Ischämien, cerebralen Infarkten, Psychosen bedingt durch erhöhten Aminosäurenspiegel, Schlaganfällen, Hirnödemen, Hypoxie, Anoxie, AIDS-Demens, Encephalomyelitis, Tourette-Syndrom, Tinnitus aurium und perinataler Asphyxie.

## Claims

1. A medicament containing at least one substituted 2-heteroarylaminoacetic acid compound of the general formula I, 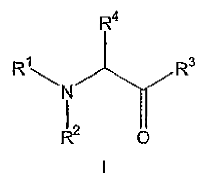 in which
R¹ denotes a monocyclic aryl or heteroaryl residue, which may be at least monosubstituted and/or fused with an optionally at least monosubstituted monocyclic or polycyclic ring system optionally comprising at least one heteroatom as a ring member, wherein
the corresponding substituents are, in each case mutually independently, selected from the group consisting of linear or branched, optionally at least monosubstituted, C₁₋₆ alkyl optionally attached via an oxygen atom or a sulfur atom, linear or branched, optionally at least monosubstituted C₂₋₆ alkenyl, optionally attached via an oxygen atom or a sulfur atom, linear or branched, optionally at least monosubstituted, C₂₋₆ alkynyl optionally attached via an oxygen atom or a sulfur atom, optionally at least monosubstituted C₃₋₈ cycloalkyl optionally comprising at least one heteroatom, optionally attached via an oxygen atom or a sulfur atom, halogen, OH, SH, CN, optionally at least monosubstituted phenyl, naphthyl, furanyl, thiophenyl, pyrrolyl, imidazolyl, pyrazolyl, pyridinyl, pyrimidinyl, quinolinyl or isoquinolinyl optionally attached via an oxygen atom or a sulfur atom, wherein if one of the above-stated substituents is itself mono- or polysubstituted, the substituents thereof are selected from the group consisting of F, Cl, Br, OH, CN, CF₃, CHF₂ and CH₂F.
R² denotes hydrogen or a branched or unbranched, optionally at least monounsaturated, optionally at least monosubstituted aliphatic residue, wherein
the substituents thereof are selected from the group consisting of F, Cl, Br, OH and CN,
R³ denotes the group OR⁵, SR⁵ or NR⁵R⁶, wherein
R⁵ and optionally R⁶ mutually independently in each case denote hydrogen, a branched or unbranched, optionally at least monounsaturated, optionally at least monosubstituted aliphatic residue, a saturated or unsaturated, optionally at least monosubstituted cycloaliphatic residue optionally comprising at least one heteroatom as a ring member, or an optionally at least monosubstituted aryl or heteroaryl residue, wherein
if R⁵ and/or R⁶ denote(s) an at least monosubstituted, branched or unbranched, optionally at least monounsaturated aliphatic residue, the substituents thereof may preferably be selected from the group consisting of F, Cl, Br, OH and CN,
if R⁵ and/or R⁶ denote(s) an at least monosubstituted, saturated or unsaturated cycloaliphatic residue optionally comprising at least one heteroatom as a ring member, the substituents thereof may preferably be selected from the group consisting of F, Cl, Br, OH, CN, CF₃, CHF₂, CH₂F, OCF₃, OCHF₂ and OCH₂F,
if R⁵ and/or R⁶ denote(s) an at least monosubstituted aryl or heteroaryl residue, the substituents thereof may preferably be selected from the group consisting of F, Cl, Br, OH, CN, CF₃, CHF₂, CH₂F, OCF₃, OCHF₂ and OCH₂F,
and
R⁴ denotes an optionally at least monosubstituted benzofuran or benzothiophene residue, wherein,
the corresponding substituents are, in each case mutually independently, selected from the group consisting of halogen, CₓF₂ₓ₊ᵢ (x = integer from 1-6), CN, OR⁷, SR⁷, CO₂R⁷, CON(R⁷)₂, N(R⁷)₂, optionally at least monosubstituted C₃₋₈ cycloalkyl, optionally at least monosubstituted, branched or unbranched C₁₋₆ alkyl, optionally at least monosubstituted, branched or unbranched C₂₋₆ alkenyl, optionally at least monosubstituted, branched or unbranched C₂₋₆ alkynyl and optionally at least monosubstituted phenyl, naphthyl, furanyl, thiophenyl, pyrrolyl, imidazolyl, pyrazolyl, pyridinyl, pyrimidinyl, quinolinyl or isoquinolinyl, wherein
the residue R⁷ is selected from the group consisting of hydrogen, optionally at least monosubstituted C₃₋₈ cycloalkyl, optionally at least monosubstituted, branched or unbranched C₁₋₆ alkyl, optionally at least monosubstituted, branched or unbranched C₂₋₆ alkenyl, optionally at least monosubstituted, branched or unbranched C₂₋₆ alkynyl and optionally at least monosubstituted phenyl, naphthyl, furanyl, thiophenyl, pyrrolyl, imidazolyl, pyrazolyl, pyridinyl, pyrimidinyl, quinolinyl or isoquinolinyl, wherein
if one of these above-stated aromatic or heteroaromatic substituents is itself mono- or polysubstituted, the substituents thereof may preferably be selected from the group consisting of F, Cl, Br, OH, CN, CF₃, CHF₂, CH₂F, OCF₃, OCHF₂ and OCH₂F,
if one of these above-stated aliphatic or cycloaliphatic substituents is itself mono- or polysubstituted, the substituents thereof may preferably be selected from the group consisting of F, Cl, Br, OH and CN,
optionally in the form of one of the pure stereoisomers thereof, in particular enantiomers or diastereomers, the racemate thereof or in the form of a mixture of stereoisomers, in particular the enantiomers or diastereomers, in any desired mixing ratio, or in each case in the form of the acid thereof or the base thereof or in the form of the physiologically acceptable salt thereof, in particular sodium salt or hydrochloride salt, or in each case in the form of the solvate thereof, in particular hydrate.

2. A medicament according to claim 1, **characterised in that** the residue R¹, denotes a 5- or 6-membered, monocyclic aryl or heteroaryl residue,
which may be at least monosubstituted and/or fused with an optionally at least monosubstituted, mono-, di- or tricyclic ring system optionally comprising at least one heteroatom as a ring member, wherein the rings of the ring system are in each case 5-to 7-membered, preferably denotes an optionally at least monosubstituted phenyl, naphthyl, furanyl, thiophenyl, pyrrolyl, imidazolyl, pyrazolyl, pyridinyl, pyrimidinyl, quinolinyl or isoquinolinyl residue, particularly preferably denotes a phenyl residue which is unsubstituted or preferably identically substituted in 3,5 position,
wherein
the corresponding substituents are, in each case mutually independently, selected from the group consisting of linear or branched, optionally at least monosubstituted, C₁₋₆ alkyl optionally attached via an oxygen atom or a sulfur atom, linear or branched,
optionally at least monosubstituted C₂₋₆ alkenyl, optionally attached via an oxygen atom or a sulfur atom, linear or branched,
optionally at least monosubstituted, C₂₋₆ alkynyl optionally attached via an oxygen atom or a sulfur atom, optionally at least monosubstituted C₃₋₈ cycloalkyl optionally comprising at least one heteroatom, optionally attached via an oxygen atom or a sulfur atom, halogen, OH, SH, CN, optionally at least monosubstituted phenyl, naphthyl, furanyl, thiophenyl, pyrrolyl, imidazolyl, pyrazolyl, pyridinyl, pyrimidinyl, quinolinyl or isoquinolinyl optionally attached via an oxygen atom or a sulfur atom, wherein
if one of the above-stated substituents is itself mono- or polysubstituted, the substituents thereof are selected from the group consisting of F, Cl, Br, OH, CN, CF₃, CHF₂ and CH₂F.

3. A medicament according to claim 1 or claim 2, **characterised in that** the residue R² denotes hydrogen or a branched or unbranched, optionally at least monounsaturated, optionally at least monosubstituted aliphatic C₁₋₃ residue, preferably hydrogen, wherein
the substituents thereof are selected from the group consisting of F, Cl, Br, OH and CN.

4. A medicament according to any one of claims 1 to 3, **characterised in that** the residue R³ denotes the group OR⁵ or NR⁵R⁶, preferably the group OR⁵.

5. A medicament according to any one of claims 1 to 4, **characterised in that** the residues R⁵ and optionally R⁶ mutually independently in each case denote hydrogen, a branched or unbranched, optionally at least monounsaturated, optionally at least monosubstituted aliphatic C₁₋₆ residue, a saturated or unsaturated, optionally at least monosubstituted cycloaliphatic C₃₋₈ residue optionally comprising at least one heteroatom as a ring member, or a 5- or 6-membered, optionally at least monosubstituted aryl or heteroaryl residue, preferably hydrogen or an optionally at least monosubstituted aliphatic C₁₋₆ residue, particularly preferably hydrogen, wherein
if R⁵ and/or R⁶ denote(s) an at least monosubstituted, branched or unbranched, optionally at least monounsaturated aliphatic residue, the substituents thereof may preferably be selected from the group consisting of F, Cl, Br, OH and CN,
if R⁵ and/or R⁶ denote(s) an at least monosubstituted, saturated or unsaturated cycloaliphatic residue optionally comprising at least one heteroatom as a ring member, the substituents thereof may preferably be selected from the group consisting of F, Cl, Br, OH, CN, CF₃, CHF₂, CH₂F, OCF₃, OCHF₂ and OCH₂F,
if R⁵ and/or R⁶ denote(s) an at least monosubstituted aryl or heteroaryl residue, the substituents thereof may preferably be selected from the group consisting of F, Cl, Br, OH, CN, CF₃, CHF₂, CH₂F, OCF₃, OCHF₂ and OCH₂F.

6. A medicament according to any one of claims 1 to 5, **characterised in that** the residue R⁴ denotes an at least monosubstituted benzofuran or benzothiophene residue, preferably a residue selected from the group consisting of benzo[b]furan-2-yl, benzo[b]furan-3-yl, benzo[b]thiophen-2-yl and benzo[b]thiophen-3-yl, in each case unsubstituted or at least monosubstituted, wherein
the corresponding substituents are, in each case mutually independently, selected from the group consisting of halogen, CₓF₂ₓ₊₁ (x = integer from 1-6), CN, OR⁷, SR⁷, CO₂R⁷, CON(R⁷)₂, N(R⁷)₂, optionally at least monosubstituted C₃₋₈ cycloalkyl, optionally at least monosubstituted, branched or unbranched C₁₋₆ alkyl, optionally at least monosubstituted, branched or unbranched C₂₋₆ alkenyl, optionally at least monosubstituted, branched or unbranched C₂₋₆ alkynyl and optionally at least monosubstituted phenyl, naphthyl, furanyl, thiophenyl, pyrrolyl, imidazolyl, pyrazolyl, pyridinyl, pyrimidinyl, quinolinyl or isoquinolinyl, wherein
the residue R⁷ is selected from the group consisting of hydrogen, optionally at least monosubstituted C₃₋₈ cycloalkyl, optionally at least monosubstituted, branched or unbranched C₁₋₆ alkyl, optionally at least monosubstituted, branched or unbranched C₂₋₆ alkenyl, optionally at least monosubstituted, branched or unbranched C₂₋₆ alkynyl and optionally at least monosubstituted phenyl, naphthyl, furanyl, thiophenyl, pyrrolyl, imidazolyl, pyrazolyl, pyridinyl, pyrimidinyl, quinolinyl or isoquinolinyl, wherein
if one of these above-stated aromatic or heteroaromatic substituents is itself mono- or polysubstituted, the substituents thereof may preferably be selected from the group consisting of F, Cl, Br, OH, CN, CF₃, CHF₂, CH₂F, OCF₃, OCHF₂ and OCH₂F,
if one of these above-stated aliphatic or cycloaliphatic substituents is itself mono- or polysubstituted, the substituents thereof may preferably be selected from the group consisting of F, Cl, Br, OH and CN.

7. A medicament according to any one of claims 1 to 6, **characterised in that** the compound of the general formula I is selected from the group consisting of:
benzo[b]furan-2-yl-(3,5-dichlorophenylamino)acetic acid, benzo[b]furan-2-yl-(3,5-bis(trifluoromethyl)phenylamino)acetic acid,
benzo[b]thiophen-2-yl-(3,5-dichlorophenylamino)acetic acid, benzo[b]thiophen-3-yl-(3,5-dichlorophenylamino)acetic acid and benzo[b]thiophen-3-yl-(3,5-bis(trifluoromethyl)phenylaminoacetic acid,
optionally in the form of one of the pure stereoisomers thereof, in particular enantiomers or diastereomers, the racemate thereof or in the form of a mixture of stereoisomers, in particular the enantiomers or diastereomers, in any desired mixing ratio, or in each case in the form of the acid thereof or the base thereof or in the form of the physiologically acceptable salt thereof, in particular sodium salt or hydrochloride salt, or in each case in the form of the solvate thereof, in particular hydrate.

8. A medicament according to one or more of claims 1 to 7 for combatting pain.

9. A medicament according to claim 8 for combatting chronic pain.

10. A medicament according to claim 8 for combatting neuropathic pain.

11. A medicament according to one or more of claims 1 to 7 for anxiolysis.

12. A medicament according to one or more of claims 1 to 7 for the treatment of inflammatory and/or allergic reactions.

13. A medicament according to one or more of claims 1 to 7 for the treatment of depression.

14. A medicament according to one or more of claims 1 to 7 for the treatment of the abuse of drugs and/or medicines and/or alcohol.

15. A medicament according to one or more of claims 1 to 7 for the treatment of gastritis.

16. A medicament according to one or more of claims 1 to 7 for the treatment of diarrhoea.

17. A medicament according to one or more of claims 1 to 7 for the treatment of urinary incontinence.

18. A medicament according to one or more of claims 1 to 7 for the treatment of cardiovascular diseases.

19. A medicament according to one or more of claims 1 to 7 for the treatment of airways diseases.

20. A medicament according to one or more of claims 1 to 7 for the treatment of coughing.

21. A medicament according to one or more of claims 1 to 7 for the treatment of mental health conditions.

22. A medicament according to one or more of claims 1 to 7 for the treatment of epilepsy.

23. A medicament according to one or more of claims 1 to 7 for the treatment of schizophrenia.

24. A medicament according to one or more of claims 1 to 7 for the treatment of neurodegenerative diseases, preferably Alzheimer's disease and/or Huntington's chorea and/or Parkinson's disease and/or multiple sclerosis.

25. A medicament according to one or more of claims 1 to 7 for the treatment of cerebral ischaemic episodes.

26. A medicament according to one or more of claims 1 to 7 for the treatment of cerebral infarcts.

27. A medicament according to one or more of claims 1 to 7 for the treatment of psychoses brought about by elevated amino acid levels.

28. A medicament according to one or more of claims 1 to 7 for the treatment of strokes.

29. A medicament according to one or more of claims 1 to 7 for the treatment of cerebral oedema.

30. A medicament according to one or more of claims 1 to 7 for the treatment of hypoxia.

31. A medicament according to one or more of claims 1 to 7 for the treatment of anoxia.

32. A medicament according to one or more of claims 1 to 7 for the treatment of AIDS dementia.

33. A medicament according to one or more of claims 1 to 7 for the treatment of encephalomyelitis.

34. A medicament according to one or more of claims 1 to 7 for the treatment of Tourette's syndrome.

35. A medicament according to one or more of claims 1 to 7 for the treatment of tinnitus.

36. A medicament according to one or more of claims 1 to 7 for the treatment of perinatal asphyxia.

37. Use of at least one substituted 2-heteroarylaminoacetic acid compound according to one or more of claims 1 to 7 for the production of a medicament for the treatment of pain, preferably of chronic pain and/or neuropathic pain.

38. Use of at least one substituted 2-heteroarylaminoacetic acid compound according to one or more of claims 1 to 7 for the production of a medicament for the treatment of anxiety states, inflammatory and/or allergic reactions, depression, abuse of drugs and/or medicines and/or alcohol, gastritis, diarrhoea, urinary incontinence, cardiovascular diseases, airways diseases, coughing, mental health conditions, epilepsy, schizophrenia, neurodegenerative diseases, preferably Alzheimer's disease and/or Huntington's chorea and/or Parkinson's disease and/or multiple sclerosis, cerebral ischaemic episodes, cerebral infarcts, psychoses brought about by elevated amino acid levels, strokes, cerebral oedema, hypoxia, anoxia, AIDS dementia, encephalomyelitis, Tourette's syndrome, tinnitus and perinatal asphyxia.

## Revendications

1. Médicament contenant au moins un composé substitué d'acide 2-hétéroarylaminoacétique de formule générale I, 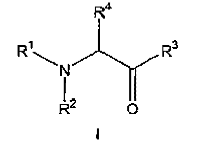 où
R¹ représente un radical aryle ou hétéroaryle monocyclique, qui peut être au moins monosubstitué et/ou condensé avec un système monocyclique ou polycyclique le cas échéant au moins monosubstitué, présentant le cas échéant au moins un hétéroatome comme élément de cycle, où
les substituants correspondantes, à chaque fois indépendamment l'un de l'autre, sont choisis dans le groupe constitué par C₁₋₆- alkyle linéaire ou ramifié, le cas échéant au moins monosubstitué, le cas échéant lié via un atome d'oxygène ou un atome de soufre, C₂₋ ₆-alcényle linéaire ou ramifié, le cas échéant au moins monosubstitué, le cas échéant lié via un atome d'oxygène ou un atome de soufre, C₂₋₆-alcynyle linéaire ou ramifié, le cas échéant au moins monosubstitué, le cas échéant lié via un atome d'oxygène ou un atome de soufre, C₃₋₈-cycloalkyle le cas échéant au moins monosubstitué, présentant le cas échéant au moins un hétéroatome, le cas échéant lié via un atome d'oxygène ou un atome de soufre, halogène, OH, SH, CN ; phényle, naphtyle, furannyle, thiophényle, pyrrolyle, imidazolyle, pyrazolyle, pyridinyle, pyrimidinyle, quinoléinyle ou isoquinoléinyle le cas échéant au moins monosubstitué, le cas échéant lié via un atome d'oxygène ou un atome de soufre, où
pour autant qu'un de ces substituants susmentionnés soit lui-même monosubstitué ou polysubstitué, ses substituants sont choisis dans le groupe constitué par F, Cl, Br, OH, CN, CF₃, CHF₂ et CH₂F,
R² représente hydrogène ou un radical aliphatique ramifié ou non ramifié, le cas échéant au moins monoinsaturé, le cas échéant au moins monosubstitué, où
ses substituant sont choisis dans le groupe constitué par F, Cl, Br, OH et CN,
R³ représente le groupe OR⁵, SR⁵ ou NR⁵R⁶, où
R⁵ et le cas échéant R⁶, indépendamment l'un de l'autre, représente(nt) à chaque fois hydrogène, un radical aliphatique ramifié ou non ramifié, le cas échéant au moins monoinsaturé, le cas échéant au moins monosubstitué ou un radical cycloaliphatique saturé ou insaturé, présentant le cas échéant au moins un hétéroatome comme élément de cycle, le cas échéant au moins monosubstitué ou un radical aryle ou hétéroaryle le cas échéant au moins monosubstitué, où
- pour autant que R⁵ et/ou R⁶ représentent un radical aliphatique au moins monosubstitué, ramifié ou non ramifié, le cas échéant au moins monoinsaturé, leurs substituants sont choisis dans le groupe constitué par F, Cl, Br, OH et CN,
- pour autant que R⁵ et/ou R⁶ représentent un radical cycloaliphatique au moins monosubstitué, saturé ou insaturé, présentant le cas échéant au moins un hétéroatomc comme élément de cycle, leurs substituants sont choisis dans le groupe constitué par F, Cl, Br, OH, CN, CF₃, CHF₂, CH₂F, OCF₃, OCHF₂ et OCH₂F,
- pour autant que R⁵ et/ou R⁶ représentent un radical aryle ou hétéroaryle au moins monosubstitué, leurs substituants sont choisis dans le groupe constitué par F, Cl, Br, OH, CN, CF₃, CHF₂, CH₂F, OCF₃, OCHF₂ et OCH₂F,
et
R⁴ représente un radical benzofuranne ou benzothiophène le cas échéant au moins monosubstitué, où
les substituants correspondants, à chaque fois indépendamment l'un de l'autre, sont choisis dans le groupe constitué par halogène, CₓF₂ₓ₊₁(x = nombre entier de 1-6), CN, OR⁷, SR⁷, CO₂R⁷, CON(R⁷)₂, N(R⁷)₂, C₃₋₈- cycloalkyle le cas échéant au moins monosubstitué, C₁₋₆-alkyle le cas échéant au moins monosubstitué, ramifié ou non ramifié, C₂₋₆-alcényle le cas échéant au moins monosubstitué, ramifié ou non ramifié, C₂₋₆- alcynyle le cas échéant au moins monosubstitué, ramifié ou non ramifié ; et phényle, naphtyle, furannyle, thiophényle, pyrrolyle, imidazolyle, pyrazolyle, pyridinyle, pyrimidinyle, quinoléinyle ou isoquinoléinyle le cas échéant au moins monosubstitué, où
le radical R⁷ est choisi dans le groupe constitué par hydrogène, C₃₋₈-cycloalkyle le cas échéant au moins monosubstitué, C₁₋₆-alkyle le cas échéant au moins monosubstitué, ramifié ou non ramifié, C₂₋₆-alcénylé le cas échéant au moins monosubstitué, ramifié ou non ramifié, C₂₋₆-alcynyle le cas échéant au moins monosubstitué, ramifié ou non ramifié ; et phényle, naphtyle, furannyle, thiophényle, pyrrolyle, imidazolyle, pyrazolyle, pyridinyle, pyrimidinyle, quinoléinyle ou isoquinoléinyle le cas échéant au moins monosubstitué, où
- pour autant qu'un de ces substituants aromatiques ou hétéroaromatiques susmentionnés soit lui-même monosubstitué ou polysubstitué, ses substituants sont choisis dans le groupe constitué par F, Cl, Br, OH, CN, CF₃, CHF₂, CH₂F, OCF₃, OCHF₂ et OCH₂F,
- pour autant qu'un de ces substituants aliphatiques ou cycloaliphatiques susmentionnés soit lui-même monosubstitué ou polysubstitué, ses substituants sont choisis dans le groupe constitué par F, Cl, Br, OH et CN,
le cas échéant sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de ses acides ou de ses bases ou sous forme de ses sels physiologiquement acceptables, en particulier le sel de sodium ou chlorhydrate ou à chaque fois sous forme de ses solvates, en particulier les hydrates.

2. Médicament selon la revendication 1,
**caractérisé**
**en ce que** le radical R¹ représente un radical aryle ou hétéroaryle de 5 ou 6 chaînons, monocyclique, qui peut être au moins monosubstitué et/ou condensé avec un système monocyclique, bicyclique ou tricyclique le cas échéant au moins monosubstitué, présentant le cas échéant au moins un hétéroatome comme élément de cycle, les cycles du système cyclique étant à chaque fois de 5 à 7 chaînons, de préférence un radical phényle, naphtyle, furannyle, thiophényle, pyrrolyle, imidazolyle, pyrazolyle, pyridinyle, pyrimidinyle, quinoléinyle ou isoquinoléinyle, le cas échéant au moins monosubstitué, de manière particulièrement préférée un radical phényle non substitué ou substitué en position 3,5, de préférence de manière identique, où
les substituants correspondants, à chaque fois indépendamment l'un de l'autre, sont choisis dans le groupe constitué par C₁₋₆-alkyle linéaire ou ramifié, le cas échéant au moins monosubstitué, le cas échéant lié via un atome d'oxygène ou un atome de soufre, C₂₋₆-alcényle linéaire ou ramifié, le cas échéant au moins monosubstitué, le cas échéant lié via un atome d'oxygène ou un atome de soufre, C₂₋₆-alcynyle linéaire ou ramifié, le cas échéant au moins monosubstitué, le cas échéant lié via un atome d'oxygène ou un atome de soufre, C₃₋₈-cycloalkyle le cas échéant au moins monosubstitué, présentant le cas échéant au moins un hétéroatome, le cas échéant lié via un atome d'oxygène ou un atome de soufre, halogène, OH, SH, CN ; phényle, naphtyle, furannyle, thiophényle, pyrrolyle, imidazolyle, pyrazolyle, pyridinyle, pyrimidinyle, quinoléinyle ou isoquinoléinyle le cas échéant au moins monosubstitué, le cas échéant lié via un atome d'oxygène ou un atome de soufre, où
pour autant qu'un de ces substituants susmentionnés soit lui-même monosubstitué ou polysubstitué, ses subslituants sont choisis dans le groupe constitué par F, Cl, Br, OH, CN, CF₃, CHF₂ et CH₂F.

3. Médicament selon la revendication 1 ou 2,
**caractérisé**
**en ce que** le radical R² représente hydrogène ou un radical aliphatique en C₁₋₃ ramifié ou non ramifié, le cas échéant au moins monoinsaturé, le cas échéant au moins monosubstitué, de préférence hydrogène, où
ses substituants sont choisis dans le groupe constitué par F, Cl, Br, OH et CN.

4. Médicament selon l'une quelconque des revendications 1 à 3, **caractérisé**
**en ce que** le radical R³ représente le groupe OR⁵ ou NR⁵R⁶, de préférence le groupe OR⁵.

5. Médicament selon l'une quelconque des revendications 1 à 4, **caractérisé**
**en ce que** les radicaux R⁵ et le cas échéant R⁶, indépendamment l'un de l'autre, représentent à chaque fois hydrogène, un radical aliphatique en C₁₋₆ ramifié ou non ramifié, le cas échéant au moins monoinsaturé, le cas échéant au moins monosubstitué, un radical cycloaliphatique en C₃₋₈ saturé ou insaturé, présentant le cas échéant au moins un hétéroatome comme élément de cycle, le cas échéant au moins monosubstitué ou un radical aryle ou hétéroaryle de 5 ou 6 chaînons, le cas échéant au moins monosubstitué, de préférence hydrogène ou un radical aliphatique en C₁₋₆ le cas échéant au moins monosubstitué, de manière particulièrement préférée hydrogène, où
- pour autant que R⁵ et/ou R⁶ représentent un radical aliphatique au moins monosubstitué, ramifié ou non ramifié, le cas échéant au moins monoinsaturé, leurs substituants sont choisis dans le groupe constitué par F, Cl, Br, OH et CN,
pour autant que R⁵ et/ou R⁶ représentent un radical cycloaliphatique au moins monosubstitué, saturé ou insaturé, présentant le cas échéant au moins un hétéroatome comme élément de cycle, leurs substituants sont choisis dans le groupe constitué par F, Cl, Br, OH, CN, CF₃, CHF₂, CH₂F, OCF₃, OCHF₂ et OCH₂F,
- pour autant que R⁵ et/ou R⁶ représentent un radical aryle ou hétéroaryle au moins monosubstitué, leurs substituants sont choisis dans le groupe constitué par F, Cl, Br, OH, CN, CF₃, CHF₂, CH₂F, OCF₃, OCHF₂ et OCH₂F.

6. Médicament selon l'une quelconque des revendications 1 à 5, **caractérisé**
**en ce que** le radical R⁴ représente un radical choisi dans le groupe constitué par benzo[b]furann-2-yle, benzo[b]furann-3-yle, benzo[b]thiophén-2-yle et benzo[b]thiophén-3-yle, à chaque fois non substitué ou au moins monosubstitué, où
- les substituants correspondants, à chaque fois indépendamment l'un de l'autre, sont choisis dans le groupe constitué par halogène, CₓF₂ₓ₊₁(x = nombre entier de 1-6), CN, OR⁷, SR⁷, CO₂R⁷, CON(R⁷)₂, N(R⁷)₂, C₃₋₈-cycloalkyle le cas échéant au moins monosubstitué, C₁₋₆-alkyle le cas échéant au moins monosubstitué, ramifié ou non ramifié, C₂₋₆-alcényle le cas échéant au moins monosubslitué, ramifié ou non ramifié, C₂₋₆-alcynyle le cas échéant au moins monosubstitué, ramifié ou non ramifié ; et phényle, naphtyle, furannyle, thiophényle, pyrrolyle, imidazolyle, pyrazolyle, pyridinyle, pyrimidinyle, quinoléinyle ou isoquinoléinyle le cas échéant au moins monosubstitué, où
- le radical R⁷ est choisi dans le groupe constitué par hydrogène, C₃₋₈-cycloalkyle le cas échéant au moins monosubstitué, C₁₋₆-alkyle le cas échéant au moins monosubstitué, ramifié ou non ramifié, C₂₋₆-alcényle le cas échéant au moins monosubstitué, ramifié ou non ramifié, C₂₋₆-alcynyle le cas échéant au moins monosubstitué, ramifié ou non ramifié ; et phényle, naphtyle, furannyle, thiophényle, pyrrolyle, imidazolyle, pyrazolyle, pyridinyle, pyrimidinyle, quinoléinyle ou isoquinoléinyle le cas échéant au moins monosubstitué, où
- pour autant qu'un de ces substituants aromatiques ou hétéroaromatiques susmentionnés soit lui-même monosubstitué ou polysubstitué, ses substituants peuvent de préférence être choisis dans le groupe constitué par F, Cl, Br, OH, CN, CF₃, CHF₂, CH₂F, OCF₃, OCHF₂ et OCH₂F,
- pour autant qu'un de ces substituants aliphatiques ou cycloaliphatiques susmentionnés soit lui-même monosubstitué ou polysubstitué, ses substituants peuvent de préférence être choisis dans le groupe constitué par F, Cl, Br, OH et CN.

7. Médicament selon l'une quelconque des revendications 1 à 6, **caractérisé**
**en ce que** le composé de formule générale I est choisi dans le groupe constitué par :
- l'acide benzo[b]furann-2-yl-(3,5-dichlorophénylamino)-acétique,
- l'acide benzo[b]furann-2-yl-(3,5-bis(trifluorométhyl)phénylamino)-acétique,
- l'acide benzo[b]thiophén-2-yl-(3,5-dichlorophénylamino)-acétique,
- l'acide benzo[b]thiophén-3-yl-(3,5-dichlorophénylamino)-acétique et
- l'acide benzo[b]thiophén-2-yl-(3,5-bis(trifluorométhyl)phénylaminoacétique,
le cas échéant sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de ses acides ou de ses bases ou sous forme de ses sels physiologiquement acceptables, en particulier le sel de sodium ou chlorhydrate ou à chaque fois sous forme de ses solvates, en particulier les hydrates.

8. Médicament selon l'une ou plusieurs des revendications 1 à 7 destiné à lutter contre les douleurs.

9. Médicament selon la revendication 8 destiné à lutter contre des douleurs chroniques.

10. Médicament selon la revendication 8 destiné à lutter contre des douleurs neuropathiques.

11. Médicament selon l'une ou plusieurs des revendications 1 à 7 destiné à l'anxiolyse.

12. Médicament selon l'une ou plusieurs des revendications 1 à 7 destiné au traitement de réactions inflammatoires et/ou allergiques.

13. Médicament selon l'une ou plusieurs des revendications 1 à 7 destiné au traitement de dépressions.

14. Médicament selon l'une ou plusieurs des revendications 1 à 7 destiné au traitement lors d'un abus de drogues et/ou de médicaments et/ou d'alcool.

15. Médicament selon l'une ou plusieurs des revendications 1 à 7 destiné au traitement de la gastrite.

16. Médicament selon l'une ou plusieurs des revendications 1 à 7 destiné au traitement de la diarrhée.

17. Médicament selon l'une ou plusieurs des revendications 1 à 7 destiné au traitement de l'incontinence urinaire.

18. Médicament selon l'une ou plusieurs des revendications 1 à 7 destiné au traitement de maladies cardiovasculaires.

19. Médicament selon l'une ou plusieurs des revendications 1 à 7 destiné au traitement de maladies des voies respiratoires.

20. Médicament selon l'une ou plusieurs des revendications 1 à 7 destiné au traitement de la toux.

21. Médicament selon l'une ou plusieurs des revendications 1 à 7 destiné au traitement de maladies psychiques.

22. Médicament selon l'une ou plusieurs des revendications 1 à 7 destiné au traitement de l'épilepsie.

23. Médicament selon l'une ou plusieurs des revendications 1 à 7 destiné au traitement de la schizophrénie.

24. Médicament selon l'une ou plusieurs des revendications 1 à 7 destiné au traitement de maladies de neurodégénérescence, de préférence de la maladie d'Alzheimer et/ou de Huntington et/ou de Parkinson et/ou de la sclérose en plaques.

25. Médicament selon l'une ou plusieurs des revendications 1 à 7 destiné au traitement d'ischémies cérébrales.

26. Médicament selon l'une ou plusieurs des revendications 1 à 7 destiné au traitement d'infarctus cérébraux.

27. Médicament selon l'une ou plusieurs des revendications 1 à 7 destiné au traitement de psychoses provoquées par un niveau élevé d'acides aminés.

28. Médicament selon l'une ou plusieurs des revendications 1 à 7 destiné au traitement d'attaques.

29. Médicament selon l'une ou plusieurs des revendications 1 à 7 destiné au traitement d'oedèmes cérébraux.

30. Médicament selon l'une ou plusieurs des revendications 1 à 7 destiné au traitement de l'hypoxie.

31. Médicament selon l'une ou plusieurs des revendications 1 à 7 destiné au traitement de l'anoxie.

32. Médicament selon l'une ou plusieurs des revendications 1 à 7 destiné au traitement de la démence liée au SIDA.

33. Médicament selon l'une ou plusieurs des revendications 1 à 7 destiné au traitement de l'encéphalomyélite.

34. Médicament selon l'une ou plusieurs des revendications 1 à 7 destiné au traitement du syndrome de Gilles de la Tourette.

35. Médicament selon l'une ou plusieurs des revendications 1 à 7 destiné au traitement du Tinnitus aurium (acouphènes).

36. Médicament selon l'une ou plusieurs des revendications 1 à 7 destiné au traitement lors de l'asphyxie périnatale.

37. Utilisation d'au moins un composé substitué de l'acide 2-hétéroarylaminoacétique selon l'une ou plusieurs des revendications 1 à 7 pour la préparation d'un médicament destiné au traitement de douleurs, de préférence de douleurs chroniques et/ou de douleurs neuropathiques.

38. Utilisation d'au moins un composé selon l'une ou plusieurs des revendications 1 à 7 pour la préparation d'un médicament destiné au traitement d'états d'anxiété, de réactions inflammatoires et/ou allergiques, de dépressions, d'abus de drogues et/ou de médicaments et/ou d'alcool, de la gastrite, de la diarrhée, de l'incontinence urinaire, de maladies cardiovasculaires, de maladies des voies respiratoires, de la toux, de maladies psychiques, de l'épilepsie, de la schizophrénie, de maladies de neurodégénérescence, de préférence de la maladie d'Alzheimer et/ou de Huntington et/ou de Parkinson et/ou de la sclérose en plaques, d'ischémies cérébrales, d'infarctus cérébraux, de psychoses provoquées par un niveau élevé d'acides aminés, d'attaques, d'oedèmes cérébraux, de l'hypoxie, de l'anoxie, de la démence liée au SIDA, de l'encéphalomyélite, du syndrome de Gilles de la Tourette, du Tinnitus aurium (acouphènes) et de l'asphyxie périnatale.
